(19) Europäisches Patentamt
European Patent Office
Office européen des brevets

(11) EP 2 344 913 B1

(12) **EUROPEAN PATENT SPECIFICATION**

(45) Date of publication and mention of the grant of the patent:
**18.02.2015 Bulletin 2015/08**

(21) Application number: **09817361.0**

(22) Date of filing: **30.09.2009**

(51) Int Cl.:
***G01N 21/62*** *(2006.01)* ***G01N 33/68*** *(2006.01)*

(86) International application number:
**PCT/IL2009/000937**

(87) International publication number:
**WO 2010/038228 (08.04.2010 Gazette 2010/14)**

(54) **METHOD AND SYSTEM FOR EMITTING LIGHT**

VERFAHREN UND SYSTEM ZUR LICHTEMISSION

PROCÉDÉ ET SYSTÈME D'ÉMISSION DE LUMIÈRE

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **02.10.2008 US 136785 P**
**01.04.2009 US 202759 P**

(43) Date of publication of application:
**20.07.2011 Bulletin 2011/29**

(73) Proprietor: **Ramot at Tel-Aviv University Ltd.
61392 Tel Aviv (IL)**

(72) Inventors:
- **AMDURSKY, Nadav
53305 Givataim (IL)**
- **GAZIT, Ehud
47264 Ramat-HaSharon (IL)**
- **ROSENMAN, Gil
75770 Rishon-Le Zion (IL)**

(74) Representative: **Dennemeyer & Associates S.A.
55, rue des Bruyères
1274 Howald (LU)**

(56) References cited:
**WO-A1-2004/060791 WO-A1-2005/090386
WO-A1-2007/116069 WO-A2-03/030821
WO-A2-2007/038600 US-A1- 2002 042 121
US-A1- 2005 214 222 US-A1- 2005 214 380
US-A1- 2006 098 194 US-A1- 2006 228 300
US-A1- 2006 256 338 US-A1- 2007 157 325
US-A1- 2007 157 325 US-A1- 2008 009 434
US-A1- 2008 009 434 US-A1- 2009 117 609
US-A1- 2010 053 928 US-B1- 6 803 188**

- **HIDEYUKI TANAKA ET AL: "Enhanced Current Efficiency from Bio-Organic Light-Emitting Diodes Using Decorated Amyloid Fibrils with Conjugated Polymer", NANO LETTERS, ACS, US, vol. 8, no. 9, 10 September 2008 (2008-09-10), pages 2858-2861, XP002634279, ISSN: 1530-6984, DOI: 10.1021/NL801510Z [retrieved on 2008-08-12]**
- **NADAV AMDURSKY ET AL: "Blue Luminescence Based on Quantum Confinement at Peptide Nanotubes", NANO LETTERS, vol. 9, no. 9, 9 September 2009 (2009-09-09), pages 3111-3115, XP55020819, ISSN: 1530-6984, DOI: 10.1021/nl9008265**
- **AMDURSKY N ET AL: "Self-assembled bioinspired quantum dots: Optical properties", APPLIED PHYSICS LETTERS, AIP, AMERICAN INSTITUTE OF PHYSICS, MELVILLE, NY, US, vol. 94, no. 26, 2 July 2009 (2009-07-02), pages 261907-261907, XP012121851, ISSN: 0003-6951, DOI: 10.1063/1.3167354**
- **DUKES K D ET AL: "Monitoring the earliest amyloid-beta oligomers via quantized photobleaching of dye-labeled peptides", ANALYTICAL BIOCHEMISTRY, ACADEMIC PRESS INC, NEW YORK, vol. 382, no. 1, 19 July 2008 (2008-07-19) , pages 29-34, XP025408772, ISSN: 0003-2697, DOI: 10.1016/J.AB.2008.07.011 [retrieved on 2008-07-19]**
- **"Amyloid plaque", Genetics Home Reference, 5 December 2008 (2008-12-05), XP55021299, Retrieved from the Internet: URL:http: //web.archive.org/web/20081216085 040/http: //ghr.nlm.nih.gov/glossary=amyloi dplaque [retrieved on 2012-03-08]**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

**(Cont. next page)**

- "Luminescence", Wikipedia, 14 August 2008 (2008-08-14), XP55021220, Online Retrieved from the Internet: URL:http://en.wikipedia.org/w/index.php?title=Luminescence&oldid=231964227&printable=yes [retrieved on 2012-03-07]
- TANAKA ET AL.: 'Enhanced Current Efficiency from Bio-Organic Light-Emitting Diodes Using Decorated Amyloid Fibrils with Conjugated Polymer.' NANO LETTERS, [Online] vol. 8, no. 9, 12 August 2008, pages 2858 - 2861, XP002634279 Retrieved from the Internet: <URL:http://pubs.acs.org/doi/abs/10.1021/nl 801510z> [retrieved on 2010-10-01]
- KIM ET AL.: 'Two-dimensional infrared spectra of isotopically diluted amyloid fibrils from A-40.' PNAS USA, [Online] vol. 105, no. 22, 03 June 2008, pages 7720 - 7725, XP008146318 Retrieved from the Internet: <URL:http://www.pnas.org/content/105/22/772 0.abstract> [retrieved on 2010-10-01]
- NILSSON ET AL. IMAGING DISTINCT CONFORMATIONAL STATES OF AMYLOID-FIBRILS IN ALZHEIMER'S DISEASE USING NOVEL LUMINESCENT PROBES., [Online] vol. 2, no. 8, 2007, pages 553 - 560, XP003026386 Retrieved from the Internet: <URL:http://pubs.acs.org/doi/abs/10.1021/cb 700116u> [retrieved on 2010-10-01]
- STABO-EEG ET AL.: 'Quantum efficiency and two-photon absorption cross-section of conjugated polyelectrolytes used for protein conformation measurements with applications on amyloid structures.' CHEMICAL PHYSICS, [Online] vol. 336, no. 2-3, 2007, pages 121 - 126., XP022164725 Retrieved from the Internet: <URL: http://www.sciencedirect.com> [retrieved on 2010-10-01]
- JI ET AL.: 'An alternative approach to amyloid fibrils morphology: CdSe/ZnS quantum dots labelled beta-amyloid peptide fragments Abeta (31-35), Abeta(1?40) and Abeta(1-42).' COLLOIDS AND SURFACES B: BIOINTERFACES, [Online] vol. 50, no. 2, 2006, pages 104 - 111, XP027996383 Retrieved from the Internet: <URL:http: //www.sciencedirect.com>
- HELMCHEN ET AL.: 'Deep tissue two-photon microscopy.' NATURE METHODS, [Online] vol. 2, no. 12, 2005, pages 932 - 940, XP009141325 Retrieved from the Internet: <URL:http: //www.nature.com/nmeth/journal/v2 /n12/abs/nmeth818.html> [retrieved on 2010-10-01]
- PARK ET AL.: 'Polymer/Gold Nanoparticle Nanocomposite Light-Emitting Diodes: Enhancement of Electroluminescence Stability and Quantum Efficiency of Blue-Light-Emitting Polymers.' CHEMISTRY OF MATERIALS, [Online] vol. 16, no. 4, 2004, pages 688 - 692, XP002407594 Retrieved from the Internet: <URL:http://pubs.acs.org/doi/abs/10.1021/cm 0304142> [retrieved on 2010-10-01]
- VOROPAI ET AL.: 'Spectral Properties of Thioflavin T and its Complexes with Amyloid Fibrils.' JOURNAL OF APPLIED SPECTROSCOPY, [Online] vol. 7, no. 6, 2003, pages 868 - 874, XP008146319 Retrieved from the Internet: <URL:http://www.springerlink.com/content/r1 68205164013328> [retrieved on 2010-10-01]
- ANTZUTKIN ET AL.: 'Multiple quantum solid-state NMR indicates a parallel, not antiparallel, organization of beta-sheets in Alzheimer's beta-amyloid fibrils.' PNAS USA, [Online] vol. 97, no. 24, 2000, pages 13045 - 13050., XP008146320 Retrieved from the Internet: <URL:http: //www.pnas.org/content/97/24/1304 5.full> [retrieved on 2010-10-01]
- AGRANOVICH ET AL.: 'Quantum confinement and superradiance of one-dimensional self-trapped Frenkel excitons.' CHEMICAL PHYSICS, [Online] vol. 245, no. 1-3, 1999, pages 175 - 184., XP008146321 Retrieved from the Internet: <URL: http://www.sciencedirect.com/science/j ournal/03010104> [retrieved on 2010-10-01]
- WEBB ET AL.: 'Single Molecule Spectroscopy Illuminating the Molecular Dynamics of Life.' SPRINGER SERIES IN CHEMICAL PHYSICS, [Online] vol. 96, 2009, pages 107 - 117., XP008146343 Retrieved from the Internet: <URL: http://www.springerlink.com/content/01 72-6218> [retrieved on 2010-10-01]
- FRIEDRICH RALF P ET AL: "Mechanism of amyloid plaque formation suggests an intracellular basis of Abeta pathogenicity.", PROCEEDINGS OF THE NATIONAL ACADEMY OF SCIENCES OF THE UNITED STATES OF AMERICA 2 FEB 2010, vol. 107, no. 5, 2 February 2010 (2010-02-02), pages 1942-1947, ISSN: 1091-6490

## Description

RELATED APPLICATIONS

This application claims the benefit of priority from U.S. Patent Application No. 61/136,785 filed on October 2, 2008 and 61/202,758 filed on April 1, 2009.

FIELD AND BACKGROUND OF THE INVENTION

The present invention, in some embodiments thereof, relates to nanotechnology and, more particularly, but not exclusively, to a method and system for emitting light as defined in the appended claims.

Some embodiments of the present invention relate to a method for predicting formation of an amyloid plaque as defined in the appended claims.

Electronic industries have entered into a new domain of small devices dictated by quantum mechanical effects. In bulk semiconductors, charge carriers are free to move in three dimensions as their movement is not restricted by potential wells. However, useful effects may arise if the carrier motion is confined to dimensions which are sufficiently small so that quantum effects are no longer non-negligible important. This is because the carrier's mean free path is comparable or even lager that the physical dimensions of the device, and the dynamic is governed by the wave nature of the carrier. The effect is known as quantum confinement (QC) effect, and structures demonstrating such effect are known as QC structures.

The most common example of QC structure is two-dimensional QC structure, also known as a quantum well (QW) structure. In QW structures, the carriers are free to move in two dimensions, but quantum effects are significant in the third dimension. That is, the energy levels are quantized in one dimension but form continua in the other two dimensions. Also known are structures in which carrier movement is further restricted, e.g., a structure in which carriers can move freely in one dimension (one-dimensional QC, also known as quantum wire) or in which their positions are essentially localized (zero-dimensional QC, also known as quantum dot).

One type of quantum well structure is a GaAs based structure, such as a GaAs/AlGaAs structure illustrated in FIG. 1. In these quantum well structures, a double heterostructure consisting of a thin layer of GaAs (about 10 nm in thickness) whose bulk semiconductors due to the change in allowable energy levels caused by the presence of quantum effects.

Quantum confinement effect has also been reported [L. T. Canham, (1990), "Silicon quantum wire array fabrication by electrochemical and chemical dissolution of wafers," 1046-1048, AIP] in mesoporous silicon layers. It was shown that by increasing the porous size, hence decreasing the size of the bulk Si skeleton between the porous, a quantum confinement effect can take place.

Recently, quantum confinement structures were fabricated in inorganic nanorods of ZnO [Park et al., (2003) "Quantum confinement observed in ZnO/ZnMgO nanorod heterostructures," Adv. Mater. 15, 526-529].

Tanaka et al, Nano Letters, 2008, 8(9):2S58-2861, teach the use of self-assembled bionanostructures in polymer light-emitting diodes. Amyloid fibrils formed by protein misfolding are decorated with a soluble luminescent conjugated polymer, and used as the active layer in a light emitting diode, resulting in a 10-fold increase in external quantum efficiency compared with pristine polymer, because of improved carrier injection.

Dukes et al, Analytical Biochemistry, 2008, 382(1):29-34, teaches direct determination of reproducible distributions of peptide species by exploiting quantized photobleaching from individual dye-labeled Abeta peptides to characterize monomers and small oligomers tethered to the surface of functionalized coverslips.

SUMMARY OF THE INVENTION

The present invention provides a light emitting system according to claim 1, a method of emitting light according to claim 2, and a method of predicting formation of an amyloid plaque in a peptide sample according to claim 3.

According to an aspect of some embodiments of the present invention there is provided a method of predicting formation of an amyloid plaque in a peptide sample. The method comprises determining presence of quantum confinement in the sample, and predicting that formation of an amyloid plaque is likely to occur if the sample exhibits quantum confinement. The method may further comprise issuing a report regarding the prediction.

According to some embodiments of the invention the amount of soluble peptides in the peptide sample is at least 2 times higher than an amount of insoluble peptides in the solution.

According to some embodiments of the invention the peptide sample is substantially devoid of insoluble peptides.

According to some embodiments of the invention the determination is by measuring optical absorption spectrum.

According to some embodiments of the invention the quantum confinement is manifested as a step-like shape of the optical absorption spectrum.

According to some embodiments of the invention the determination is by measuring a photoluminescence excitation spectrum.

According to some embodiments of the invention the quantum confinement is manifested as a sufficiently narrow peak in the photoluminescence excitation spectrum. According to some embodiments of the invention the peak is has a full-width-at-half-maximum (FWHM) of less than 20 nm or less than 10 nm, *e.g.,* 5 nm.

According to some embodiments of the invention the photoluminescence excitation spectrum is measured at several concentrations, wherein the sufficiently narrow peak is a concentration-dependent peak.

According to some embodiments of the invention the sufficiently narrow peak is between a wavelength of 280 nm and a wavelength of 295 nm.

According to some embodiments of the invention the sample contains insulin, wherein the determination is by measuring a photoluminescence excitation spectrum, and wherein the quantum confinement is manifested as a sufficiently narrow peak between a wavelength of 280 nm and a wavelength of 295 nm.

According to an aspect of some embodiments of the present invention there is provided a light emitting system. The system comprises a plurality of peptide nanostructures forming crystalline structures which exhibit quantum confinement, and means for exciting the peptide nanostructures to emit light, wherein said means is selected from the group consisting of:a pair of electrodes configured to excite said sub-nanometric crystalline structures by injecting holes and electrons to said peptide nanostructures, thereby to emit light via injection luminescence; a heat source configured to excite said sub-nanometric crystalline structures by heating said peptide nanostructures, thereby to emit light via thermoluminescence; and means which comprises or is connectable to a voltage source configured to excite said sub-nanometric crystalline structures by applying voltage to said peptide nanostructures, thereby to emit light via electroluminescence.

According to an aspect of some embodiments of the present invention there is provided a method of emitting light. The method comprises exciting a plurality of peptide nanostructures forming organic sub-nanometric crystalline structure which exhibits quantum confinement, so as to emit light, wherein said exciting is selected from the group consisting of using a voltage source for generating electric field for exciting said sub-nanometric crystalline structure to emit light via electroluminescence, using a pair of electrodes for injecting holes and electrons to said sub-nanometric crystalline structure to emit light via injection luminescence, and heating said sub-nanometric crystalline structure to emit light via thermoluminescence.

According to some embodiments of the invention the crystalline structure is a two-dimensional quantum confinement structure.

According to some embodiments of the invention the crystalline structure is a zero-dimensional quantum confinement structure.

According to some embodiments of the invention the crystalline structure is a sub-nanometric crystalline structure.

According to some embodiments of the invention the system is configured for two-photon emission.

According to an aspect of some embodiments of the present disclosure there is provided a laser system. The laser system comprises a light emitting system as delineated above and optionally as further detailed hereinunder.

According to an aspect of some embodiments of the present disclosure there is provided a display system. The display system comprises a light emitting system as delineated above and optionally as further detailed hereinunder.

According to an aspect of some embodiments of the present disclosure there is provided an optical communication system. The communication system comprises a light emitting system as delineated above and optionally as further detailed hereinunder.

According to an aspect of some embodiments of the present disclosure there is provided an illumination system. The illumination system comprises a light emitting system as delineated above and optionally as further detailed hereinunder.

According to an aspect of some embodiments of the present disclosure there is provided an optical connector system. The optical connector system comprises a light emitting system as delineated above and optionally as further detailed hereinunder.

According to an aspect of some embodiments of the present disclosure there is provided a system for analyzing a target material. The system comprises a light emitting system as delineated above and optionally as further detailed hereinunder.

According to an aspect of some embodiments of the present disclosure there is provided an imaging system. The imaging system comprises a light emitting system as delineated above and optionally as further detailed hereinunder.

According to an aspect of some embodiments of the present disclosure there is provided a quantum teleportation system. The quantum teleportation system comprises a light emitting system as delineated above and optionally as further detailed hereinunder.

According to an aspect of some embodiments of the present disclosure there is provided a quantum cryptography system. The quantum cryptography system comprises a light emitting system as delineated above and optionally as

further detailed hereinunder.

According to an aspect of some embodiments of the present disclosure there is provided a quantum computer system. The quantum computer system comprises a light emitting system as delineated above and optionally as further detailed hereinunder.

Unless otherwise defined, all technical and/or scientific terms used herein have the same meaning as commonly understood by one of ordinary skill in the art to which the invention pertains. Although methods and materials similar or equivalent to those described herein can be used in the practice or testing of embodiments of the invention, exemplary methods and/or materials are described below. In case of conflict, the patent specification, including definitions, will control. In addition, the materials, methods, and examples are illustrative only and are not intended to be necessarily limiting.

Implementation of the method and/or system of embodiments of the invention can involve performing or completing selected tasks manually, automatically, or a combination thereof. Moreover, according to actual instrumentation and equipment of embodiments of the method and/or system of the invention, several selected tasks could be implemented by hardware, by software or by firmware or by a combination thereof using an operating system.

For example, hardware for performing selected tasks according to embodiments of the invention could be implemented as a chip or a circuit. As software, selected tasks according to embodiments of the invention could be implemented as a plurality of software instructions being executed by a computer using any suitable operating system. In an exemplary embodiment of the invention, one or more tasks according to exemplary embodiments of method and/or system as described herein are performed by a data processor, such as a computing platform for executing a plurality of instructions. Optionally, the data processor includes a volatile memory for storing instructions and/or data and/or a non-volatile storage, for example, a magnetic hard-disk and/or removable media, for storing instructions and/or data. Optionally, a network connection is provided as well. A display and/or a user input device such as a keyboard or mouse are optionally provided as well.

## BRIEF DESCRIPTION OF THE DRAWINGS

Some embodiments of the invention are herein described, by way of example only, with reference to the accompanying drawings and images. With specific reference now to the drawings in detail, it is stressed that the particulars shown are by way of example and for purposes of illustrative discussion of embodiments of the invention. In this regard, the description taken with the drawings makes apparent to those skilled in the art how embodiments of the invention may be practiced.

In the drawings:

FIG. 1 is a schematic illustration of a GaAs/AlGaAs quantum well structure.

FIGS. 2A-C are schematic illustrations if a light emitting system, according to various exemplary embodiments of the present invention.

FIGS. 3A-D are schematic illustrations density-of-states plots as a function of the energy.

FIG. 4 is a schematic illustration of a utility system, according to various exemplary embodiments of the present invention.

FIGS. 5A-C are schematic illustrations of a system for analyzing a target material by two photon absorption, according to some embodiments of the present invention.

FIG. 6 is a schematic illustration of a communication system, according to some embodiments of the present invention.

FIG. 7 is a schematic illustration of a quantum computer system, according to some embodiments of the present invention.

FIG. 8 which is a flowchart diagram of a method suitable for predicting formation of an amyloid plaque in a peptide sample, according to various exemplary embodiments of the present invention.

FIG. 9 shows absorption spectrum of vapor deposited FF peptide nanotubes (black line and left scale) and FF monomers in aqueous solution (red line and right scale), as measured in an experiment performed according to some embodiments of the present invention.

FIG. 10A shows absorption spectra of Fmoc-FF hydrogel at several concentrations, as measured in an experiment performed according to some embodiments of the present invention.

FIG. 10B show absorption spectra of Fmoc-2-Nal hydrogel at several concentrations, as measured in an experiment performed according to some embodiments of the present invention.

FIG. 11 shows absorption spectrum of Fmoc-FF nanospheres at several concentrations, as measured in an experiment performed according to some embodiments of the present invention.

FIG. 12 shows photoluminescence emission, excitation and absorption spectra of vapor deposition FF peptide nanotubes, as measured in an experiment performed according to some embodiments of the present invention.

FIG. 13A shows excitation spectrum of Fmoc-FF hydrogel at various concentrations, as measured in an experiment performed according to some embodiments of the present invention. The emission wavelength is at 325 nm.

FIG. 13B shows excitation spectrum of Fmoc-2-Nal hydrogel at various concentrations, as measured in an experiment performed according to some embodiments of the present invention. The emission wavelength is at 345 nm.

FIG. 14 shows excitation spectrum of Fmoc-FF nanospheres at various concentrations, as measured in an experiment performed according to some embodiments of the present invention. The emission wavelength is 317 nm.

FIGS. 15A and 15B show photoluminescence spectrum of Fmoc-FF hydrogel at several concentrations and at two excitation wavelengths of 270 nm (FIG. 15A) and 310 nm (FIG. 15B), as measured in an experiment performed according to some embodiments of the present invention.

FIGS. 16A and 16B show photoluminescence spectrum of Fmoc-FF nanospheres in DMSO at excitation wavelengths of 300 nm (FIG. 16A) and 308 nm (FIG. 16B), as measured in an experiment performed according to some embodiments of the present invention.

FIG. 17 shows optical absorption of FF peptide nanotubes (solid line) and FF monomers (dash line), as measured in an experiment performed according to some embodiments of the present invention.

FIG. 18 shows photoluminescence (black curve) and optical absorption (red curve) spectra of normally aligned peptide nanotubes, as measured in an experiment performed according to some embodiments of the present invention. The excitation wavelength for the photoluminescence emission measurements was 260 nm.

FIG. 19A shows photoluminescence spectrum of FF peptide nanostructures as measured in experiments performed according to some embodiments of the present invention for the detection of the emission at 450 nm (solid line) and 305 nm (dashed line).

FIG. 19B shows photoluminescence spectrum of FF peptide nanostructures at two excitation wavelengths, at 370 nm (solid line) and at 260 nm (dashed line), as measured in experiments performed according to some embodiments of the present invention.

FIG. 20 is a fluorescence microscopy image of a patterned surface of FF peptide nanostructures on silicon under excitation at 340-380 nm. The blue squares are the photoluminescence emission from the FF peptide nanostructures, the purple circle is the reflections from surface of the excitation beam.

FIGS. 21A and 21B show absorption spectra of peptide nanospheres (FIG. 21A) and unordered structures (FIG. 21B) for three concentrations, as measured in experiments performed according to some embodiments of the present invention.

FIGS. 22A and 22B shows photoluminescence excitation spectra of peptide nanospheres (FIG. 22A) and the unordered structures (FIG. 22B) at several concentrations, as measured in experiments performed according to some embodiments of the present invention. The emission wavelength is 282 nm

FIGS. 23A and 23B shows photoluminescence spectrum of peptide nanospheres at concentrations of 4 mg/ml (red solid line) and 1 mg/ml (black dashed line) at excitation wavelengths of 270 nm (FIG. 23A) and 255 nm (FIG. 23B), as measured in experiments performed according to some embodiments of the present invention. The photoluminescence excitation spectrum and the Stokes shift (15 nm) are shown in FIG. 23A.

FIGS. 23C and 23D shows photoluminescence spectrum of unordered structures at 4 mg/ml (red solid line) and 1 mg/ml (black dashed line) at excitation wavelengths of 270 nm (FIG. 23A) and 255 nm (FIG. 23B), as measured in experiments performed according to some embodiments of the present invention.

FIG. 24A is an AFM image of insulin fibrils.

FIG. 24B shows a cross-section of two insulin fibrils along the line marked by block arrow in FIG. 24A.

FIG. 25A shows absorption spectrum of 0.5 mg/ml insulin, at 0 and 2 hours from the preparation of the solution, as measured in experiments performed according to some embodiments of the present invention.

FIG. 25B shows photoluminescence excitation spectrum of insulin, as measured in experiments performed according to some embodiments of the present invention, 0 hours from the preparation of the solution. The excitation spectrum was measured at emission wavelength of 305 nm.

## DESCRIPTION OF SPECIFIC EMBODIMENTS OF THE INVENTION

The present invention, in some embodiments thereof, relates to nanotechnology and, more particularly, but not exclusively, to a method and system for emitting light as defined in the appended claims.

Some embodiments of the present invention relate to a method for predicting formation of an amyloid plaque as defined in the appended claims.

Before explaining at least one embodiment of the invention in detail, it is to be understood that the invention is not necessarily limited in its application to the details of construction and the arrangement of the components and/or methods set forth in the following description and/or illustrated in the drawings and/or the Examples. The invention is capable of other embodiments or of being practiced or carried out in various ways.

Heretofore, quantum confinement effect has only been reported in inorganic structures, through fabrication of

quantum wells, wires and dots by conventional microelectronic technology. The present inventors discovered the existence of quantum confinement effect in organic materials. Based in this discovery, the present inventors have devised method and system for emitting light from an organic material, and a technique for predicting crystallization in biological substances.

Referring now to the drawings, FIGS. 2A-C are schematic illustrations of a light emitting system 10, according to various exemplary embodiments of the present invention.

System 10 comprises a plurality of peptide nanostructures 12 forming a crystalline structure which exhibits quantum confinement.

The term "peptide" as used herein encompasses native peptides (either degradation products, synthetically synthesized peptides or recombinant peptides) and peptidomimetics (typically, synthetically synthesized peptides), as well as peptoids and semipeptoids which are peptide analogs, which may have, for example, modifications rendering the peptides more stable while in a body. Such modifications include, but are not limited to N terminus modification, C terminus modification, peptide bond modification, including, but not limited to, $CH_2$-NH, $CH_2$-S, $CH_2$-S=O, O=C-NH, $CH_2$-O, $CH_2$-$CH_2$, S=C-NH, CH=CH or CF=CH, backbone modifications, and residue modification. Methods for preparing peptidomimetic compounds are well known in the art and are specified, for example, in Quantitative Drug Design, C.A. Ramsden Gd., Chapter 17.2, F. Choplin Pergamon Press (1992), Further details in this respect are provided hereinunder.

Peptide bonds (-CO-NH-) within the peptide may be substituted, for example, by N-methylated bonds (-N($CH_3$)-CO-), ester bonds (-C(R)H-C-O-O-C(R)-N-), ketomethylen bonds (-CO-$CH_2$-), $\alpha$-aza bonds (-NH-N(R)-CO-), wherein R is any alkyl, e.g., methyl, carba bonds (-$CH_2$-NH-), hydroxyethylene bonds (-CH(OH)-$CH_2$-), thioamide bonds (-CS-NH-), olefinic double bonds (-CH=CH-), retro amide bonds (-NH-CO-), peptide derivatives (-N(R)-$CH_2$-CO-), wherein R is the "normal" side chain, naturally presented on the carbon atom. These modifications can occur at any of the bonds along the peptide chain and even at several (2-3) at the same time.

The term "crystalline structure," as used herein, refers to a three dimensional ordered arrangement of atoms or molecules, which possesses symmetry characteristics. The ordering of the atoms or molecules is manifested by an elementary lattice unit, (also known as a "unit cell") having definite faces that intersect at definite angles, and possesses one or more symmetry characteristics which are described mathematically by a symmetry group (also known as the "crystallographic point group"). The overall structure of the crystal is periodic, namely, it possesses a translational symmetry, and the elementary lattice unit defines the periodicity of the crystal. The symmetry group that describes the symmetry characteristics of the crystal is referred to as a "space group", and is defined as the combination of the symmetry group that describes the translational symmetry with the crystallographic point group. A crystalline structure and its space group can be experimentally identified by means of X-ray crystallography as is well known to those skilled in the art of crystallography. A crystalline structure can also be detected by means of measuring spectral absorption, as further detailed hereinunder.

The term "quantum confinement," as used herein refers to a phenomenon in which there are quantized energy levels in at least one dimension.

A structure (such as peptide nanostructures **12**) exhibits quantum confinement when the positions of charge carriers (electrons or holes) in the structure are confined along at least one dimension. A structure in which the charge carriers are confined along one dimension but are free to move in the other two dimensions is referred to herein as a "two-dimensional quantum confinement structure," since the structure allows free motion in two dimensions. A structure in which the charge carriers are confined along two dimensions but and are free to move only in one dimension is referred to herein as a "one-dimensional quantum confinement structure," since the structure allows free motion in one dimension. A structure in which the charge carriers are confined along all three dimensions, namely a structure in which the charge carriers are localized, is referred to herein as a "zero-dimensional quantum confinement structure," since the structure does not allow free motion.

A two-dimensional quantum confinement structure is interchangeably referred to herein as a quantum well structure, a one-dimensional quantum confinement structure is interchangeably referred to herein as a quantum wire structure, and a zero-dimensional quantum confinement structure is interchangeably referred to herein as a quantum dot structure.

In various exemplary embodiments of the invention the length $L_{QC}$ of the smallest dimension along which a quantum confinement occurs is in the nanometer range, preferably below 3 nm or below 2 nm. In some embodiments of the present invention $L_{QC}$ is in the sub-nanometer range (*i.e.*, less than 1 nm), preferably less than 0.8 nm or less than 0.7 nm or less than 0.6 nm. This is an advantageous over traditional inorganic semiconductor quantum confinement structure which posses much higher quantum confinement lengths. $L_{QC}$ is referred to as the quantum confinement length.

Quantum confinement can be verified by examining the optical properties of the structure. For quantum confinement structures, the optical properties are significantly different from other structures since the optical absorption coefficient is defined by density of states (DOS) of the charge carriers. For a structure which does not exhibits any quantum confinement the DOS is proportional to the square root of the energy. For a quantum confinement structure, the DOS quantized. Representative illustrations of DOS plots as a function of the energy are provided in FIGS. 3A-D,

where FIG. 3A illustrate a smooth DOS behavior which is characteristic to a structure which does not exhibits any quantum confinement, FIG. 3B illustrates a step-like DOS behavior which is characteristic to a quantum well structure, FIG. 3C illustrates a tooth-like DOS behavior which is characteristic to a quantum wire structure and FIG. 3D illustrates a spike-like DOS behavior which is characteristic to a quantum dot structure.

Thus, when the absorption spectrum of a structure has a step-like shape, the structure can be identified as a quantum well structure.

A step-like shape of a spectrum is a widely used term in the scientific community and a person ordinarily skilled in the art of spectral analysis would recognize a spectrum having a step-like shape by observing a plot of the absorption coefficient as a function of the wavelength. Typically, but not exclusively, a step-like spectrum is characterized by a change of at least 10 % in the absorption coefficient over a wavelength range of less than 10 nm.

When the absorption spectrum of a structure has a spike-like shape, the structure can be identified as a quantum dot structure.

A spike-like shape of a spectrum is a widely used term in the scientific community and a person ordinarily skilled in the art of spectral analysis would recognize a spectrum having a spike-like shape by observing a plot of the absorption coefficient as a function of the wavelength. A spike-like spectrum is characterized by at least one peak in the absorption coefficient. Typically, but not exclusively, the width of a peak in a spike-like spectrum, as measured at half of the peak's height above the base of the peak, is less than 10 nm.

The quantum confinement length $L_{QC}$ can be estimated from the optical properties of the nanostructures using an appropriate calculation model. Representative examples for such estimation are provided in the Examples section that follows.

Peptide nanostructures suitable for the present embodiments are described in International Patent Publication Nos. WO2004/052773, WO2004/060791, WO2005/000589, WO2006/027780, WO2006/013552, WO2006/013552, WO2008/068752 and WO2009/034566, all assigned to the same assignee as the present application.

The peptide nanostructures can also be provided as a component in a hydrogel material.

As used herein, "hydrogel" refers to a material that comprises nanostructures formed of water-soluble natural or synthetic polymer chains, typically containing more than 90% or more than 95% or more than 99 % water.

A hydrogel material suitable for the present embodiments is found in International Patent Publication No. WO2007/043048.

The peptides forming the nanostructures of the some embodiments of the present invention comprise from 2 to 15 amino acid residues. More preferably, the peptides are short peptides of less than 10 amino acid residues, more preferably less than 8 amino acid residues and more preferably are peptides of 2-6 amino acid residues, and hence each peptide preferably has 2, 3, 4, 5, or 6 amino acid residues.

As used herein the phrase "amino acid" or "amino acids" is understood to include the 20 naturally occurring amino acids; those amino acids often modified post-translationally *in vivo,* including, for example, hydroxyproline, phosphoserine and phosphothreonine; and other unusual amino acids including, but not limited to, 2-aminoadipic acid, hydroxylysine, isodesmosine, nor-valine, nor-leucine and ornithine. Furthermore, the term "amino acid" includes both D- and L-amino acids.

Natural aromatic amino acids, Trp, Tyr and Phe, may be substituted for synthetic non-natural acid such as Phenylglycine, TIC, napthylalanine (Nal), phenylisoserine, threoninol, ring-methylated derivatives of Phe, halogenated derivatives of Phe or O-methyl-Tyr and $\beta$-amino acids.

The peptides of the present embodiments may include one or more modified amino acids or one or more non-amino acid monomers (e.g. fatty acids, complex carbohydrates etc).

The peptides utilized for forming the nanostructures of the present embodiments are typically linear peptides. Yet, cyclic forms of the peptide are not excluded from the scope of the present invention.

In some embodiments of the present invention the peptides composing the peptide nanostructures of the present embodiments comprise one or more aromatic amino acid residue. The advantage of having such peptides is that the aromatic functionalities which are built into the peptide allow the various peptide building blocks to interact through attractive aromatic interactions, to thereby form the nanostructure.

The phrase "aromatic amino acid residue", as used herein, describes an amino acid residue that has an aromatic moiety, as defined herein, in its side-chain.

Thus, according to some embodiments of the present invention, each of the peptides composing the peptide nanostructures comprises the amino acid sequence X-Y or Y-X, wherein X is an aromatic amino acid residue and Y is any other amino acid residue.

The peptides can be at least 2 amino acid in length.

In some embodiments of the present invention, one or several of the peptides forming the nanostructures is a polyaromatic peptide, which comprises two or more aromatic amino acid residues.

As used herein the phrase "polyaromatic peptides" refers to peptides which include at least 80 %, more preferably at least 85 %, more preferably at least 90 %, more preferably at least 95 % or more aromatic amino acid residues. In

some embodiments, at least one peptide consists essentially of aromatic amino acid residues. In some embodiments, each peptide consists essentially of aromatic amino acid residues.

Thus for example, the peptides used for forming the nanostructures can include any combination of: dipeptides composed of one or two aromatic amino acid residues; tripeptides including one, two or three aromatic amino acid residues; and tetrapeptides including two, three or four aromatic amino acid residues and so on.

In some embodiments of the present invention, the aromatic amino acid can be any naturally occurring or synthetic aromatic residue including, but not limited to, phenylalanine, tyrosine, tryptophan, phenylglycine, or modificants, precursors or functional aromatic portions thereof.

In some embodiments, one or more peptides in the plurality of peptides used for forming the nanostructures include two amino acid residues, and hence is a dipeptide.

In some embodiments, each of the peptides used for forming the nanostructures comprises two amino acid residues and therefore the nanostructures are formed from a plurality of dipeptides.

Each of these dipeptides can include one or two aromatic amino acid residues. Preferably, but not obligatorily each of these dipeptides includes two aromatic amino acid residues. The aromatic residues composing the dipeptide can be the same, such that the dipeptide is a homodipeptide, or different. Preferably, the nanostructures are formed from homodipeptides.

Hence, in various exemplary embodiments of the invention each peptide in the plurality of peptides used for forming the nanostructures is a homodipeptide composed of two aromatic amino acid residues that are identical with respect to their side-chains residue.

The aromatic amino acid residues used for forming the nanostructures can comprise an aromatic moiety, where the phrase "aromatic moiety" describes a monocyclic or polycyclic moiety having a completely conjugated pi-electron system. The aromatic moiety can be an all-carbon moiety or can include one or more heteroatoms such as, for example, nitrogen, sulfur or oxygen. The aromatic moiety can be substituted or unsubstituted, whereby when substituted, the substituent can be, for example, one or more of alkyl, trihaloalkyl, alkenyl, alkynyl, cycloalkyl, aryl, heteroaryl, heteroalicyclic, halo, nitro, azo, hydroxy, alkoxy, thiohydroxy, thioalkoxy, cyano and amine.

Exemplary aromatic moieties include, for example, phenyl, biphenyl, naphthalenyl, phenanthrenyl, anthracenyl, [1,10]phenanthrolinyl, indoles, thiophenes, thiazoles and, [2,2']bipyridinyl, each being optionally substituted. Thus, representative examples of aromatic moieties that can serve as the side chain within the aromatic amino acid residues described herein include, without limitation, substituted or unsubstituted naphthalenyl, substituted or unsubstituted phenanthrenyl, substituted or unsubstituted anthracenyl, substituted or unsubstituted [1,10]phenanthrolinyl, substituted or unsubstituted [2,2']bipyridinyl, substituted or unsubstituted biphenyl and substituted or unsubstituted phenyl.

The aromatic moiety can alternatively be substituted or unsubstituted heteroaryl such as, for example, indole, thiophene, imidazole, oxazole, thiazole, pyrazole, pyridine, pyrimidine, quinoline, isoquinoline, quinazoline, quinoxaline, and purine. When substituted, the phenyl, naphthalenyl or any other aromatic moiety includes one or more substituents such as, but not limited to, alkyl, trihaloalkyl, alkenyl, alkynyl, cycloalkyl, aryl, heteroaryl, heteroalicyclic, halo, nitro, azo, hydroxy, alkoxy, thiohydroxy, thioalkoxy, cyano, and amine.

Representative examples of homodipeptides that can be used to form the nanostructures of the present embodiments include, without limitation, a naphthylalanine-naphthylalanine dipeptide, phenanthrenylalanine-phenanthrenylalanine dipeptide, anthracenylalanine-anthracenylalanine dipeptide, [1,10]phenanthrolinylalanine-[1,10]phenanthrolinylalanine dipeptide, [2,2']bipyridinylalanine-[2,2']bipyridinylalanine dipeptide, (pentahalo-phenylalanine)-(pentahalo-phenylalanine) dipeptide, phenylalanine-phenylalanine dipeptide, (amino-phenylalanine)-(amino-phenylalanine) dipeptide, (dialkylamino-phenylalanine)-( dialkylamino-phenylalanine) dipeptide, (halophenylalanine)-(halophenylalanine) dipeptide, (alkoxy-phenylalanine)-(alkoxy-phenylalanine) dipeptide, (trihalomethyl-phenylalanine)-(trihalomethyl-phenylalanine) dipeptide, (4-phenyl-phenylalanine)-(4-phenyl-phenylalanine) dipeptide and (nitro-phenylalanine)-(nitro-phenylalanine) dipeptide.

According to various exemplary embodiments of the present invention the peptide nanostructures are composed from a plurality of diphenylalanine (Phe-Phe) homodipeptides.

In some embodiments of the present invention one or more peptides in the plurality of peptides used to form the nanostructures is an end-capping modified peptide.

The phrase "end-capping modified peptide", as used herein, refers to a peptide which has been modified at the N-(amine)terminus and/or at the C-(carboxyl)terminus thereof. The end-capping modification refers to the attachment of a chemical moiety to the terminus, so as to form a cap. Such a chemical moiety is referred to herein as an end-capping moiety and is typically also referred to herein and in the art, interchangeably, as a peptide protecting moiety or group.

The phrase "end-capping moiety", as used herein, refers to a moiety that when attached to the terminus of the peptide, modifies the end-capping. The end-capping modification typically results in masking the charge of the peptide terminus, and/or altering chemical features thereof, such as, hydrophobicity, hydrophilicity, reactivity, solubility and the like. Examples of moieties suitable for peptide end-capping modification can be found, for example, in Green et al., "Protective Groups in Organic Chemistry", (Wiley, second ed. 1991) and Harrison et al., "Compendium of Synthetic

Organic Methods", Vols. 1-8 (John Wiley and Sons, 1971-1996).

The use of end-capping modification, allows to control the chemical properties and charge of the nanostructures, hence also the way the peptide nanostructures of the present embodiments are assembled and/or aligned.

Changing the charge of one or both termini of one or more of the peptides may result in altering the morphology of the resulting nanostructure and/or the way the resulting nanostructure responds to, for example, an electric and/or magnetic fields.

End-capping of a peptide can be used to modify its hydrophobic/hydrophilic nature. Altering the hydrophobic/hydrophilic property of a peptide may result, for example, in altering the morphology of the resulting nanostructure and/or the aqueous solubility thereof. By selecting the percentage of the end-capping modified peptides and the nature of the end capping modification, the hydrophobicity/hydrophilicity, as well as the solubility of the nanostructure can be finely controlled. For example, the end capping modification can be selected to control adherence of nanoparticles to the wall of the nanostructures.

It was found by the present inventors that modifying the end-capping of a peptide does not abolish its capacity to self-assemble into nanostructures, similar to the nanostructures formed by unmodified peptides. The persistence of the end-capping modified peptides to form nanostructures supports the hypothesis of the present inventors according to which the dominating characteristic required to form peptides nanostructures is the aromaticity of its side-chains, and the $\pi$-stacking interactions induced thereby, as previously described in, for example WO 2004/052773 and WO 2004/060791.

It was further found by the present inventors that the aromatic nature of at least one of the end-capping of the peptide affects the morphology of the resulting nanostructure. For example, it was found that an unmodified peptide or a peptide modified with a non-aromatic end-capping moiety can self-assemble to a tubular nanostructure.

Representative examples of N-terminus end-capping moieties suitable for the present embodiments include, but are not limited to, formyl, acetyl (also denoted herein as "Ac"), trifluoroacetyl, benzyl, benzyloxycarbonyl (also denoted herein as "Cbz"), tert-butoxycarbonyl (also denoted herein as "Boc"), trimethylsilyl (also denoted "TMS"), 2-trimethylsilyl-ethanesulfonyl (also denoted "SES"), trityl and substituted trityl groups, allyloxycarbonyl, 9-fluorenylmethyloxycarbonyl (also denoted herein as "Fmoc"), and nitro-veratryloxycarbonyl ("NVOC").

Representative examples of C-terminus end-capping moieties suitable for the present embodiments are typically moieties that lead to acylation of the carboxy group at the C-terminus and include, but are not limited to, benzyl and trityl ethers as well as alkyl ethers, tetrahydropyranyl ethers, trialkylsilyl ethers, allyl ethers, monomethoxytrityl and dimethoxytrityl. Alternatively the -COOH group of the C-terminus end-capping may be modified to an amide group.

Other end-capping modifications of peptides include replacement of the amine and/or carboxyl with a different moiety, such as hydroxyl, thiol, halide, alkyl, aryl, alkoxy, aryloxy and the like, as these terms are defined herein.

In some embodiments of the present invention, all of the peptides that form the nanostructures are end-capping modified.

End-capping moieties can be further classified by their aromaticity. Thus, end-capping moieties can be aromatic or non-aromatic.

Representative examples of non-aromatic end capping moieties suitable for N-terminus modification include, without limitation, formyl, acetyl trifluoroacetyl, tert-butoxycarbonyl, trimethylsilyl, and 2-trimethylsilyl-ethanesulfonyl. Representative examples of non-aromatic end capping moieties suitable for C-terminus modification include, without limitation, amides, allyloxycarbonyl, trialkylsilyl ethers and allyl ethers.

Representative examples of aromatic end capping moieties suitable for N-terminus modification include, without limitation, fluorenylmethyloxycarbonyl (Fmoc). Representative examples of aromatic end capping moieties suitable for C-terminus modification include, without limitation, benzyl, benzyloxycarbonyl (Cbz), trityl and substituted trityl groups.

When the nanostructures of the present embodiments comprise one or more dipeptides, the dipeptides can be collectively represented by the following general Formula I:

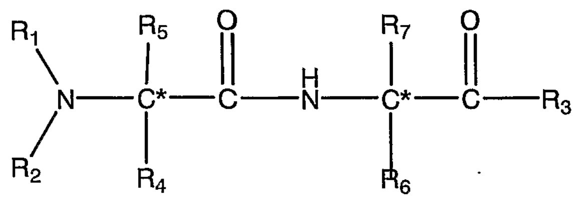

Formula I

where:

C* is a chiral carbon having a D configuration or L configuration; $R_1$ and $R_2$ are each independently selected from

the group consisting of hydrogen, alkyl, cycloalkyl, aryl, carboxy, thiocarboxy, C-carboxylate and C-thiocarboxylate; $R_3$ is selected from the group consisting of hydroxy, alkoxy, aryloxy, thiohydroxy, thioalkoxy, thioaryloxy, halo and amine; and each of $R_4$-$R_7$ is independently selected from the group consisting of hydrogen, alkyl, cycloalkyl, aryl, heteroaryl, heteroalicyclic, hydroxy, thiohydroxy (thiol), alkoxy, aryloxy, thioalkoxy, thioaryloxy, C-carboxylate, C-thiocarboxylate, N-carbamate, N-thiocarbamate, hydrazine, guanyl, and guanidine, as these terms are defined herein, provided that at least one of $R_4$-$R_7$ comprises an aromatic moiety, as defined hereinabove.

Also contemplated are embodiments in which one or more of $R_4$-$R_7$ is other substituent, provided that at least one comprises an aromatic moiety.

Also contemplated are embodiments in which one or more of $R_1$-$R_3$ is the end-capping moieties described hereinabove.

The peptide nanostructures of the present embodiments can further comprise a functional group, preferably a plurality of functional groups.

The functional group can be, for example, a group such as, but not limited to, thiol, hydroxy, halo, carboxylate, amine, amide, nitro, cyano, hydrazine, and the like, a hydrophobic moiety, such as, but not limited to, medium to high alkyls, cycloalkyls and aryls, and/or a metal ligand.

The nanostructures of the present embodiments have chemical and mechanical stability. The ability to decorate the nanostructures of the present embodiments with functional groups enables their integration into many applications.

In some embodiments of the present invention nanostructures **12** are made, at least in part from the dipeptide $NH_2$-Phe-Phe-COOH (FF).

In some embodiments of the present invention nanostructures **12** are made, at least in part, from *N*-fluorenylmethoxycarbonyl (Fmoc) based molecules, containing natural and/or unnatural aromatic amino acid.

In some embodiments of the present invention nanostructures **12** are made, at least in part, from Fmoc-Phe-Phe-OH (Fmoc-FF).

In some embodiments of the present invention nanostructures **12** are made, at least in part, from Fmoc-2-Naphtalene (Fmoc-2-Nal).

In some embodiments of the present invention nanostructures **12** are made, at least in part, from tertbutoxycarbonyl-Phe-Phe-OH (Boc-FF).

The nanostructures of the present embodiments can also incorporate additional foreign material. Such foreign material can be incorporated in more than one way. For example, when the nanostructures of the present embodiments have a tubular structure, they can be filled with a filler material. Alternatively or additionally, the nanostructures of the present embodiments can be coated at least partially by a suitable foreign material.

The nanostructures of the present embodiments may incorporate (enclose and/or be coated with) a conducting or semiconductor material, including, without limitation, inorganic structures such as Group IV, Group III/Group V, Group II/Group VI elements, transition group elements, or the like.

As used herein, the term "Group" is given its usual definition as understood by one of ordinary skill in the art. For instance, Group II elements include Zn, Cd and Hg; Group III elements include B, Al, Ga, In and Tl; Group IV elements include C, Si, Ge, Sn and Pb; Group V elements include N, P, As, Sb and Bi; and Group VI elements include O, S, Se, Te and Po.

Thus, for conducting materials, the nanostructures may incorporate, for example, silver, gold, copper, platinum, nickel, or palladium. For semiconductor materials the nanostructures may incorporate, for example, silicon, indium phosphide, gallium nitride and others.

The nanostructures may also encapsulate, for example, any organic or inorganic molecules that are polarizable or have multiple charge states. For example, the nanostructures may include main group and metal atom-based wire-like silicon, transition metal-containing wires, gallium arsenide, gallium nitride, indium phosphide, germanium, or cadmium selenide structures.

Additionally, the nanostructure may incorporate (enclose and/or be coated with) various combinations of materials, including semiconductors and dopants. Representative examples include, without limitations, silicon, germanium, tin, selenium, tellurium, boron, diamond, or phosphorous. The dopant may also be a solid solution of various elemental semiconductors, for example, a mixture of boron and carbon, a mixture of boron and P, a mixture of boron and silicon, a mixture of silicon and carbon, a mixture of silicon and germanium, a mixture of silicon and tin, or a mixture of germanium and tin. In some embodiments, the dopant or the semiconductor may include mixtures of different groups, such as, but not limited to, a mixture of a Group III and a Group V element, a mixture of Group III and Group V elements, a mixture of Group II and Group VI semiconductors. Additionally, alloys of different groups of semiconductors may also be possible, for example, a combination of a Group II-Group VI and a Group III-Group V semiconductor and a Group I and a Group VII semiconductor.

Specific and representative examples of semiconductor materials which can be encapsulated by the nanostructure of the present invention include, without limitation, CdS, CdSe, ZnS and $SiO_2$.

The nanostructure may also incorporate (enclose and/or be coated with) a thermoelectric material that exhibits a predetermined thermoelectric power. Preferably, such a material is selected so that the resulting nanostructure composition is characterized by a sufficient figure of merit. According to some embodiments of the present invention the thermoelectric material which is encapsulated in the nanostructure is a bismuth-based material, such as, but not limited to, elemental bismuth, a bismuth alloy or a bismuth intermetallic compound. The thermoelectric material may also be a mixture of any of the above materials or other materials known to have thermoelectric properties. In addition the thermoelectric material may also include a dopant. Representative examples include, without limitation, bismuth telluride, bismuth selenide, bismuth antimony telluride, bismuth selenium telluride and the like. Other materials are disclosed, for example, in U.S. Patent Application No. 20020170590.

The nanostructure may also incorporate (enclose and/or be coated with) magnetic materials, which can be diamagnetic, paramagnetic or ferromagnetic materials. Representative examples of paramagnetic materials which can be incorporated by the nanostructure include, without limitation, cobalt, copper, nickel, and platinum. Representative examples of ferromagnetic materials include, without limitation, magnetite and NdFeB.

Other materials which may be encapsulated by the nanostructure include, without limitation, light-emitting materials (*e.g.*, dysprosium, europium, terbium, ruthenium, thulium, neodymium, erbium, ytterbium or any organic complex thereof), biominerals (*e.g.,* calcium carbonate) and polymers (*e.g.,* polyethylene, polystyrene, polyvinyl chloride, polynucleotides and polypeptides).

Referring now again to FIGS. 2A-C, system **10** further comprises means **16** for exciting peptide nanostructures so as to emit light. In various exemplary embodiments of the invention nanostructures **12** emit the light at room temperature (*e.g.*, at about 15-25 °C). In some embodiments of the present invention the emission is in the ultraviolet range of wavelengths.

The present embodiments contemplate several types of means **16** for exciting the nanostructures. Generally, the type of means **16** is selected in accordance with the mechanism by which it is desired to have the light emitted from the nanostructures **12.**

FIG. 2A illustrates an embodiments of the invention in which means **16** comprises a light source **18.** In these embodiments, peptide nanostructures **12** emit light via the photoluminescence effect. Light source **18** is preferably a monochromatic light source, e.g., a laser device.

FIG. 2B illustrates an embodiments of the invention in which means **16** comprises or are connectable to a voltage source **20.** In these embodiments, peptide nanostructures **12** emit light via the electroluminescence effect. Source **20** can generate electric filed by means of electrodes **22.** Preferably, nanostructures **12** in this embodiment incorporate an electrically conductive foreign material as described above for facilitating their electrical communication with electrodes **22.** For clarity of presentation, voltage source **20** is illustrated as connected to only one of electrodes **22,** but the skilled person would appreciated that more than one electrode can be connected to source **20.** In some embodiments of the present invention, electrodes **22** injecting holes and electrons to peptide nanostructures **22,** in which case peptide nanostructures **22** emit light via injection luminescence.

The difference between the embodiment in which nanostructures **22** emit light via electroluminescence and the embodiment in which nanostructures **22** emit light via injection luminescence is, *inter alia,* in the materials from which electrodes **22** are made and/or the voltage level of source **20.** For generating light via injection luminescence, electrodes **22** are preferably made of materials having a different work function such that one electrode injects electrons and the other electrode injects holes (or equivalently receives electrons). In this embodiment the voltage source can be of relatively low voltage since it is not necessary for the generated electric field to be of high intensity. For generating light via electroluminescence, the effect is achieved primarily via application of sufficiently high electric field, in which case the electrodes can be made of the same material.

FIG. 2C illustrates an embodiments of the invention in which means **16** comprises a heat source **24.** In these embodiments, peptide nanostructures **12** emit light via the thermoluminescence effect. Preferably, nanostructures **12** in this embodiment incorporate a thermally conductive foreign material as described above for facilitating their electrical communication with heat source **24.**

In various exemplary embodiments of the invention nanostructures **12** are deposited on a substrate **14** which can be made of any material, subjected to the luminescence effect by which the nanostructures emit the light.

For example, when peptide nanostructures **12** emit light via the photoluminescence effect, substrate **14** can be made of any material, such as glass, quartz or polymeric material. In this embodiment, substrate can be made of, or being coated by, a material which reflects the light generated by light source **18.** Such construction can enhance the photo-excitation.

When peptide nanostructures **12** emit light via the electroluminescence or injection luminescence effect, substrate **14** can be made of an electrically conductive material in which case substrate **14** serves as one of the electrodes **22.** Alternatively, electrodes **22** can be deposited directly on substrate **14,** in which substrate **14** is preferably made of an electrically isolating material.

When peptide nanostructures **12** emit light via the thermoluminescence effect substrate **14** is preferably made

of a thermally conductive material so as to conduct heat from a heat source **24** to nanostructures **12.**

Peptide nanostructures **12** can be deposited on surface **14** by any technique known in the art. Representative examples include, without limitation, vapor deposition technique, wet chemistry techniques and the like. Suitable techniques for preparing and depositing peptide nanostructures are disclosed in the aforementioned international patent applications, which are assigned to the same assignee as the present application .

FIG. 4 is a schematic illustration of a utility system **40** according to various exemplary embodiments of the present invention. Utility system **40** incorporates system **10,** and various other components depending on the application for which system **40** is employed. In some embodiments, utility system **40** is a laser system, in some embodiments, utility system **40** is display system, in some embodiments, utility system **40** is an optical communication system, in some embodiments, utility system **40** is an illumination system and in some embodiments, utility system **40** is a optical connector. Such utility systems are known in the art and the skilled person would know how to construct such system using light emitting system **10** of the present embodiments.

Since nanostructures **12** or light emitting system **10** exhibit quantum confinement, system **10** can be used for two-photon emission. Two-photon emission is a process in which quantum entangled photon pairs are emitted from the system. It is recognized that quantum confinement can be produced by quantum confinement structures. In these structures, pairs of entangled photons are emitted by single photon emission from pairs of entangled electrons. A two-photon emission system is advantageous since it possesses properties absent from other emission systems.

Following are representative examples for utility system **40** which examples are particularly suitable when system **10** is a two-photon emission system. It is noted, however, that many of these examples are also applicable when system **10** does not emit entangled photons.

In an aspect of some embodiments of the present invention utility system **40** is used for two-photon microscopy, two-photon spectroscopy and/or two-photon imaging. In these embodiments the system emits two photons in the direction of a sample to induce two-photon absorption in the sample. Two-photon absorption is a process in which two distinct photons are absorbed by an ion or molecule, causing excitation from the ground state to a higher energy state to be achieved. The ion or molecule remains in the upper excited state for a short time, commonly known as the excited state lifetime, after which it relaxes back to the ground state, giving up the excess energy in the form of photons.

The use of the system of the present embodiments for microscopy and/or spectroscopy is advantageous because it allows a wider energy gap hence reduces or eliminates background photons emitted by other mechanism (e.g., infrared photons or photon emitted by thermal excitations). Thus, the two-photon emission system of the present embodiments increases signal to noise ratio.

When considering fluorescence, an important figure of merit is the quantum efficiency, defined to be the visible fluorescence intensity divided by the total input intensity. For display or spectroscopic applications based on two-photon induced fluorescence, the use of the two-photon system of the present embodiments facilitates dominance of radiative relaxation over non-radiative relaxation (phonons) hence increases the quantum efficiency.

Figs. 5A-B are schematic illustrations of a system **1000** for analyzing a target material **1002** by two photon absorption. System **1000** can be used for spectroscopy, microscopy and/or imaging of target material **1002.** For example, when target material **1002** contains a fluorophore therein, system **1000** can be used for fluorescence spectroscopy. Representative examples of fluorophores suitable for the present embodiments include fluorophores which exhibit two-photon absorption cross-sections, such as the compositions described in U.S. Patent No. 5,912,257. Also contemplated are fluorophores which are normally excitable by a single short wavelength photon (e.g., ultraviolet photon). In this embodiment, the two-photon emission system emits two long wavelength photons (e.g., infrared photons) which can be simultaneously absorbed by such fluorophores.

System **1000** comprises a two-photon emission system **1004** which emits two photons **212** and **214** in the direction of material **1002** to induce two-photon absorption therein. System **1004** can be similar to system **10** described above. Preferably, device **1004** emits photons at predetermined frequencies at frequencies $\omega_1$ and $\omega_2$. The characteristic energy diagram is illustrated in FIG. 5B showing an energy gap $\Delta E = h(\omega_1 + \omega_2)/2\pi$. Thus photons generate excitation across $\Delta E$. The value of the frequencies $\omega_1$ and $\omega_2$, is preferably selected such that $\Delta E$ is higher than the average energy of thermal and other background (e.g., infrared) photons. Once the material returns to its ground state, it emits radiation **1008** which can be detected by a detector **1006,** as known in the art. System **1000** can employ any of the components of known systems for the analysis or imaging via two-photon absorption, see, e.g., U.S. Patent Nos. 5,034,613, 6,020,591, 5,957,960, 6,267,913, 5,684,621.

Reference is now made to FIG. 5C, which is a schematic illustration of system **1000** in an embodiment in which the detection is based on two-photon absorption. In this embodiment, the optical path **1012** of photon **212** can be arranged to pass through material **1002** and the optical path **1014** of photon **214** can be arranged to bypass material **1002.** Both optical paths **1012** and **1014** terminate as detector **1006.** Thus, photon **212** can serve as a signal photon and photon **214** can serve as an idler photon.

The wavelength of photon **212** is preferably selected to allow photon **212** to excite the molecules in material **1002.** For example, the wavelength of photon **212** can be selected to match the vibrational or rotational resonances of

the molecules in the material. In biological materials, such resonances are typically in the mid infrared or far infrared. For example, most of the absorption spectra of organic compounds are generated by the vibrational overtones or the combination bands of the fundamentals of O--H, C--H, N--H, and C--C transitions. Thus, for biological materials, photon **212** can be a mid infrared photon or a far infrared photon. Also contemplated are embodiments in which photon **212** is a near infrared photon which can be suitable for molecular overtone (harmonic) and combination vibrations. The use of other wavelengths (e.g., visible photons) is not excluded from the scope of the present invention.

Optical paths **1012** and **1014** can be established via an arrangement of optical elements **1016** and **1018** such as, but not limited to, mirrors, lenses, prisms, gratings, holographic elements, graded-index optical elements, optical fibers, or other similar beam-directing mechanisms.

When signal photon **212** passes through the material, it can be either absorbed by the material giving rise to a resonance in one of the molecules or continue to propagate therethrough, with or without experiencing scattering events. If signal photon **212** is not absorbed it can continue along path **1012** to detector **1006.** Preferably optical paths **1012** and **1014** are of the same lengths such that when signal photon **212** successfully arrives at detector **1006** it arrives simultaneously with idler photon **214.**

Detector **1006** is preferably characterized by a detection threshold which equals the sum of energies of photons **212** and **214.** This can be achieved using a semiconductor detector having a sufficiently wide bandgap to allow two-photon absorption. For example, detector **1006** can be an Si detector.

Having a wide bandgap, detector **1006** does not provide a detection signal when only idler photon **214** arrives. Additionally, the wide bandgap prevents or reduces triggering of detector **1006** by noise, such as infrared background photons because the energy of such photons is lower than the detection threshold and further because triggering caused by simultaneous arrival of two background photons is extremely rare due to the random nature of the background photons.

Thus, detector **1006** provides indication of simultaneous arrival of the signal-idler photons pair, in a substantially noise-free manner. Such indication can provide information regarding material **1002** by means of transmission spectroscopy because the resonances appear as dips in the spectrum on the detector output. System **1000** can also operate according to similar principles in reflectance spectroscopy.

In an aspect of some embodiments of the present invention utility system **40** is used for communication applications. Since the light emitting system of the present embodiments typically emits two-photons simultaneously, the existence of one photon is an indication of the existence of another photon. Thus, a communication system incorporating the device of the present embodiments can use one photon as a signal and the other photon as an idler. More specifically, such communication system can transmit one photon to a distant location and use the other photon as an indication that a transmission is being made.

FIG. 6 is a schematic illustration of a communication system **1100** according to various exemplary embodiments of the present invention. System **1100** comprises a two-photon emission system **1102** which emits two photons **212** and **214.** System **1102** can be similar to system **10** described above. Preferably, system **1102** emits photons at predetermined frequencies at frequencies $\omega_1$ and $\omega_2$. One photon (photon **212** in the present example) serves as a signal as is being transmitted over a communication channel **1104** such as an optical fiber or free air, while the other photon (photon **214** in the present example) serves as an idler and being detected by a detector for indicating that the signal has been transmitted.

Such communication system can be used for quantum cryptography and quantum teleportation.

Quantum cryptography provides security by means of physical phenomenon by the uncertainty principle of Heisenberg in the quantum theory. According to the uncertainty principle, the state of quantum will be changed once it is observed, wiretapping (observation) of communication will be inevitably detectable. This allows to take measures against the wiretapping, such as shutting down the communication upon the detection of wiretapping. Thus, quantum cryptography makes undetectable wiretapping impossible physically. Moreover, the uncertainty principle explains that it is impossible to replicate particles.

Quantum teleportation is a technique to transfer quantum information ("qubits") from one place where the photons exist to another place.

A qubit is a quantum bit, the counterpart in quantum communication and computing to the binary digit or bit of classical communication and computing. Just as a bit is the basic unit of information in a classical signal, a qubit is the basic unit of information in a quantum signal. A qubit is conventionally a system having two degenerate (e.g., of equal energy) quantum states, wherein the quantum state of the qubit can be in a superposition of the two degenerate states. The two degenerate states are also referred to as basis states, and typically denoted $|0\rangle$ and $|1\rangle$. The qubit can be in any superposition of these two degenerate states, making it fundamentally different from an ordinary digital bit.

Quantum teleportation can be used to transmit quantum information in the absence of a quantum communications channel linking the sender of the quantum information to the recipient of the quantum information. Suppose, for example, that a sender, Bob, receives a qubit $\alpha|0\rangle+ \beta|1\rangle$ where and $\alpha$ and $\beta$ are parameters on a unit circle. Bob needs to transmit to a receiver, Alice, but he does not know the value of the parameters and he can only transmit classical information over to Alice. According to the laws of quantum teleportation Bob can transmit information over a classical

channel, provided Bob and Alice agree in advance to share a Bell state generated by an entangled state source. Such entangled state source can be the two-photon emission system of the present embodiments.

Thus, the system of the present embodiments can emit photons in a quantum entangled state hence be used in quantum cryptography and quantum teleportation.

In an aspect of some embodiments of the present invention utility system **40** is used as a component in a quantum computer.

Quantum computing generally involves initializing the states of several entangled qubits, allowing these states to evolve, and reading out the states of the qubits after the evolution. N entangled qubits can define an initial state that is a combination of $2^N$ classical states. This initial state undergoes an evolution, governed by the interactions that the qubits have among themselves and with external influences, providing quantum mechanical operations that have no analogy with classical computing. The evolution of the states of N qubits defines a calculation or, in effect, $2^N$ simultaneous classical calculations (e.g., conventional calculations as in those performed using a conventional computer). Reading out the states of the qubits after evolution completely determines the results of the calculations. For example, when there are two entangled qubits, $2^2=4$ simultaneous classical calculations can be performed. Taken together, quantum superposition and entanglement create an enormously enhanced computing power. Where a 2-bit register in an ordinary computer can store only one of four binary configurations (00, 01, 10, or 11) at any given time, a 2-qubit register in a quantum computer can store all four numbers simultaneously, because each qubit represents two values. If more qubits are entangled, the increased capacity is expanded exponentially.

FIG. 7 is a schematic illustration of a quantum computer system **1200** according to various exemplary embodiments of the present invention. System **1200** comprises a two-photon emission system **1202** which emits two photons **212** and **214,** as describe above. In this embodiment, photons **212** and **214** are in entangled state. System **1202** can be similar to system **10** described above. System **1200** further comprises a calculation unit **1206** which uses the photons as entangled qubits and perform calculations as known in the art (see, e.g., U.S. Patent No. 6,605,822). In various exemplary embodiments of the invention system **1200** comprises an optical mechanism **1208** for the generation of more than two entangled photons. For example, such mechanism can receives photons **212** and **214** emitted by system **1202,** generate by reflection, refraction or diffraction two or more photons from each photon, so as to produce a plurality of entangled photons **1204.**

Also contemplated are applications in which system **40** is used as an optical amplifier, in which the energy spectrum emitted by the two-photon is sufficiently broad. The use of the two-photon emission system of the present embodiments as an optical amplifier is advantageous because the gain in two-photon amplifier, in contrast to conventional single photon lasers, is nonlinear, depending on the amplitude of the light wave. Such two-photon amplifier can also be used for pulse generation. Since the length of the pulse is a decreasing function of the gain bandwidth of the amplifier, the broad spectrum of the two-photon system of the present embodiments facilitate generation of very short pulses.

The present inventors discovered that the quantum confinement possessed by the peptide nanostructures of the present embodiments allows predicting crystallization in biological substances. In particular, it was found by the present inventor that detection of quantum confinement in a sample can predict formation of amyloid material deposition.

Amyloid is a generic term referring to abnormal extracellular and/or intracellular deposits of proteins as fibrils. Amyloid fibrils may be deposited in a variety of vital organs including brain, liver, heart, kidney, pancreas, nerve and other tissues.

Commonly recognized forms of amyloid-related diseases are primary amyloidosis, secondary amyloidosis, hemodialysis-associated amyloidosis and familial amyloidosis. Amyloid material deposition (also referred to as amyloid plaque formation) is a central feature of a variety of unrelated pathological conditions including Alzheimer's disease, prion-related encephalopathies, type II diabetes mellitus, familial amyloidosis, light-chain amyloidosis, multiple myeloma and related conditions, neuropathies, cardiomyopathies, monoclonal plasma cell dyscrasias, chronic inflammation, bovine spongiform encephalopathy (BSE), Creutzfeld-Jacob disease (CJD) and scrapie disease.

Fibrillated amyloid material is composed of a dense network of rigid, nonbranching proteinaceous fibrils of indefinite length that are about 80 to 100 Å in diameter. Amyloid fibrils contain a core structure of polypeptide chains arranged in antiparallel β-pleated sheets lying with their long axes perpendicular to the long axis of the fibril.

Amyloid is not a uniform deposit and may be composed of unrelated proteins. Approximately twenty amyloid fibril proteins have been identified *in-vivo* and correlated with specific diseases (to this end see, *e.g.,* U.S. Patent No. 5,958,883). These proteins share little or no amino acid sequence homology, however the core structure of the amyloid fibrils is essentially the same. This common core structure of amyloid fibrils and the presence of common substances in amyloid deposits suggest that data characterizing a particular form of amyloid material may also be relevant to other forms of amyloid material. Amyloid deposits do not appear to be inert *in vivo,* but rather are in a dynamic state of turnover and can even regress if the formation of fibrils is halted.

The formation of amyloid plaque is known to be a slow process, and current protocols for detecting such deposition involve the use of various enhancers such serine, threonine, asparagine and glutamine amino acids.

The present inventors found that formation of amyloid plaque can be predicted at a very early stage while the

proteins building blocks are still soluble, and preferably prior to the onset of a visually detectable amyloid fibril formation. As demonstrated in the Example section that follows, there is an ultra fast crystallization process that precedes the onset of amyloid fibril formation. The present inventors have successfully identified this crystallization by detecting quantum confinement already during the first hour of incubation of a peptide sample.

Reference is now made to FIG. 8 which is a flowchart diagram of a method **80** suitable for predicting formation of an amyloid plaque in a peptide sample, according to various exemplary embodiments of the present invention. The peptide sample is preferably a solution having soluble peptides dissolved in a solvent.

The term "amyloid plaque" as used herein refers to fibrillar amyloid.

The method begins at **81** and proceed to **82** at which the method determine whether or not the sample exhibits quantum confinement. If the method identifies quantum confinement, the method proceeds to **83** at which the method predicts that formation of an amyloid plaque is likely to occur. If the method does not identify quantum confinement, the method proceeds to **84** at which the method predicts than formation of an amyloid plaque is not likely to occur. The method continues to **85** at which a report regarding the prediction is issued. The method ends at **86.**

In various exemplary embodiments of the invention the detection of quantum confinement is performed while the amount of soluble peptides in the peptide sample is at least 2 times higher than the amount of insoluble peptides in the solution. In some embodiments of the present invention the peptide sample is substantially devoid of insoluble peptides.

The phrase "substantially devoid of insoluble peptides" means that at least 90 % by weight of the solution does not contain insoluble peptides.

The determination whether or not the sample exhibits quantum confinement can be done in more than one way.

In some embodiments, the optical absorption spectrum is measured. In these embodiments, the quantum confinement is identified based on the absorption spectrum as further detailed hereinabove (see, *e.g.,* FIGS. 3B-D). Specifically, a step-like optical absorption spectrum indicates existence of two-dimensional quantum confinement structures, a tooth-like optical absorption spectrum indicates existence of one-dimensional quantum confinement structures and a spike-like optical absorption spectrum indicates existence of zero-dimensional quantum confinement structures.

In some embodiments, the photoluminescence excitation spectrum is measured. In these embodiments, the quantum confinement is manifested as a sufficiently narrow peak (*e.g.*, full-width-at-half-maximum (FWHM) of less than 20 nm or less than 10 nm), in the photoluminescence excitation spectrum. The photoluminescence excitation spectrum can be measured at several concentrations. In this embodiment, the method can indentify quantum confinement when the sufficiently narrow peak is a concentration-dependent peak. A concentration-dependent peak is a peak whose height and/or position varies with the concentration. For example, the peak can be manifested at sufficiently high concentrations (*e.g.,* above 1 mg/ml) and be less pronounced or even absent at lower concentrations. The position of the peak, once manifested, can also vary with the concentration. For example, the position of the peak can be shifted toward higher wavelengths as the concentration is raised.

In some embodiments of the present invention the sample contains insulin. In these embodiments, the quantum confinement is manifested as a sufficiently narrow peak between a wavelength of 280 nm and a wavelength of 295 nm. In experiments performed by the present inventors, this peak was observed at insulin concentration of above 1 mg/ml. The position of this peak shifted to higher wavelengths with concentration increment. At concentration of 4 mg/ml, the position of this peak was at about 287 nm.

Without being bound to any particular theory, it is expected that that quantum confinement in other amyloid proteins will also be manifested by a sufficiently narrow *(e.g.,* FWHM of less than 20 nm or less than 15 nm or less than 10 nm, *e.g.,* about 7 nm or less) peak between a wavelength of 280 nm and a wavelength of 295 nm, with a position that is shifted to higher wavelengths with concentration increment. The present inventors discovered that such position is relatively isolated from other photoluminescence excitation peaks that may be present in a sample extracted from a subject. For example, aromatic amino acids are transparent to excitation at wavelengths of 280-285 nm.

The method of the present embodiments allows predicting formation of an amyloid plaque in a variety of peptide samples, including, without limitation, a biopsy sample, a blood sample, a serum sample, a plasma sample, a urine sample, a cerebrospinal fluid sample, a peritoneal fluid sample, a stool sample and a synovial fluid sample. Also contemplated are embodiments in which the amyloid peptide from one or more of these sources is isolated in a standard buffer such as to prevent interference of the collected spectrum with other substances. Thus, the peptide sample can be a sample of one or more isolated peptides.

The ability to screen bodily fluids as a means for predicting formation of amyloid fibrils may remove the need for invasive biopsy procedures. This is particularly useful in cases of cerebral amyloidoses, where biopsies cannot be performed (Alzheimer's, Creutzfeld-Jakob Disease, bovine spongiform encephalopathy and scrapie). The present embodiments provide the opportunity for diagnosing the disease in a very early stage, prior to the formation of a visually identifiable amount of fibrils (e.g., by means of microscopy) and prior to the onset of detectable symptoms.

The method of the present embodiments is also applicable in the agricultural industry as a means of testing livestock for the presence of amyloid-associated diseases, such as, but not limited to, bovine spongiform encephalopathy

and scrapie disease.

Thus according to an aspect of some embodiments of the present disclosure there is provided a method of diagnosis. The method comprises obtaining a sample from a subject, using method **80** as described above for predicting formation of an amyloid plaque in the sample, and diagnosing the subject as having an amyloid-associated disease based on the prediction.

Thus according to an aspect of some embodiments of the present disclosure there is provided a method of treatment, comprising, identifying a subject as having an amyloidal-associated disease as described above, and administrating the subject a therapeutically effective amount of pharmaceutical composition identified for treating or preventing an amyloid-associated disease. Representative examples of pharmaceutical compositions suitable for the present embodiments are disclosed in International Publication Nos. WO2003/063760, WO2005/000193, WO2005/027901, WO2006/006172, WO2006/018850.

The method of the present embodiments can also be utilized in an assay for uncovering potential drugs useful in prevention or disaggregation of amyloid deposits. For example, the present embodiments can be used for fast screening of test compounds, whereby it is not necessary to incubate the sample with the drug until the onset of fibrillation.

As used herein the term "about" refers to $\pm$ 10 %.

The word "exemplary" is used herein to mean "serving as an example, instance or illustration." Any embodiment described as "exemplary" is not necessarily to be construed as preferred or advantageous over other embodiments and/or to exclude the incorporation of features from other embodiments.

The word "optionally" is used herein to mean "is provided in some embodiments and not provided in other embodiments." Any particular embodiment of the invention may include a plurality of "optional" features unless such features conflict.

The terms "comprises", "comprising", "includes", "including", "having" and their conjugates mean "including but not limited to".

The term "consisting of means "including and limited to".

The term "consisting essentially of" means that the composition, method or structure may include additional ingredients, steps and/or parts, but only if the additional ingredients, steps and/or parts do not materially alter the basic and novel characteristics of the claimed composition, method or structure.

As used herein, the singular form "a", "an" and "the" include plural references unless the context clearly dictates otherwise. For example, the term "a compound" or "at least one compound" may include a plurality of compounds, including mixtures thereof.

Throughout this application, various embodiments of this invention may be presented in a range format. It should be understood that the description in range format is merely for convenience and brevity and should not be construed as an inflexible limitation on the scope of the invention. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 3, 4, 5, and 6. This applies regardless of the breadth of the range.

Whenever a numerical range is indicated herein, it is meant to include any cited numeral (fractional or integral) within the indicated range. The phrases "ranging/ranges between" a first indicate number and a second indicate number and "ranging/ranges from" a first indicate number "to" a second indicate number are used herein interchangeably and are meant to include the first and second indicated numbers and all the fractional and integral numerals therebetween.

As used herein the term "method" refers to manners, means, techniques and procedures for accomplishing a given task including, but not limited to, those manners, means, techniques and procedures either known to, or readily developed from known manners, means, techniques and procedures by practitioners of the chemical, pharmacological, biological, biochemical and medical arts.

As used herein, the term "treating" includes abrogating, substantially inhibiting, slowing or reversing the progression of a condition, substantially ameliorating clinical or aesthetical symptoms of a condition or substantially preventing the appearance of clinical or aesthetical symptoms of a condition.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable subcombination or as suitable in any other described embodiment of the invention. Certain features described in the context of various embodiments are not to be considered essential features of those embodiments, unless the embodiment is inoperative without those elements.

Various embodiments and aspects of the present invention as delineated hereinabove and as claimed in the claims section below find experimental support in the following examples.

EXAMPLES

Reference is now made to the following examples, which together with the above descriptions illustrate some embodiments of the invention in a non limiting fashion.

***Example 1***

***Exemplified Quantum Well Structures***

The core recognition motif of Aβ peptide is the diphenylalanine element. The FF dipeptide has been shown to self assemble into well ordered peptide nanotubes (PNT). These structures possess very attractive and unique properties, which distinguish them from other biological entities, such as high aspect ratio with remarkably rigid structure of 20 GPa Young modulus and their high stability at temperatures up to 300 °C and in various organic solvents. The FF PNT have a wide range of diameters, from 10 nm to more then 0.5 μm. The single X-Ray structure analysis of the FF PNT showed that the diphenylalanine monomers crystallize with hydrogen-bonded head-to-tail chains in the shape of helices with four to six peptide molecules per turn. The resulting structures have chiral hydrophilic channels with a van der Waals diameter up to 10 Å.

Another version of the process of self-assembly of very short peptide molecules led to the direction of peptide based hydrogels. The hydrogels are class of materials, which can be composed from natural or synthetic polymers. They form a 3-D scaffold that can absorb a high quantity of water (>99%). They can mimic the extra cellular matrix, having good biocompatible and biodegradable qualities which enable to support the growth of cultured cells.

FF based molecules can self assemble also to nanospheres in the presence of organic solvent. It has been shown that Boc-FF in the presence of 50 % ethanol can self assemble to stable nanospheres. Fmoc-FF can also self assemble to nanospheres in the presence of high concentration of Dimethyl sulfoxide (DMSO).

In the present example the optical properties of three type of nanostructures were examined: FF-PNT deposited by vapor deposition method, Fmoc-compounds hydrogels and FF based nanospheres. For all three different types of nanostructures a step-like optical absorption spectrum and short wavelength photoluminescence in ultraviolet region were observed, indicating effect of quantum confinement. This phenomenon is a direct evidence of self assembled sub-nano-crystalline regions.

The optical absorption of FF PNT made by the vapor deposition method, the Fmoc based hydrogels and the FF based nanospheres are presented in FIGS. 9, 10A-B and 11 respectively. The FF PNT result is compared with FF monomers in aqueous solution. At the hydrogels and nanospheres samples concentration dependant results were obtained. In all cases, the absorption spectra were step-like.

The absorption spectrum of FF PNT (FIG. 9) demonstrates two distinguish steps located in the UV spectral range at 258 and 335 nm (4.8 and 3.7 eV respectively). Furthermore, a third step appears at wavelengths of about 350-420 nm. The optical spectroscopy analyses of the hydrogels PNT (FIGS. 10A-B) show one major step located at about 308-310 nm (approximately 4.0 eV). While at the FF absorbance spectrum flat steps were obtained, here the step exhibits an apex at the end. This apex indicate on a strong excitonic effects of the structures. The optical spectroscopy of the Fmoc-FF nanospheres (FIG. 11B) shows very similar results as the Fmoc-FF hydrogel, suggesting that both of these structures contain similar sub-nano crystalline regions. The Boc-FF nanospheres (FIG. 11) exhibit a slightly different absorption curve containing two peaks followed by a step. The step is located at about 260 nm. The several peaks and the location of the step indicate the involvements of several excitons and a reduced size of the sub-nano crystalline regions, as is further discussed below.

The pronounced step-like optical absorption spectrum found in all three types of nanostructures distinctly indicates on appearance of ordered, crystallized quantum well structures. Each absorption step is responsible for electron transition between energy levels created in the quantum well. The concentration dependant measurements (FIGS. 10A-B and 11), show the dynamic quantum confinement process of crystallization *in-situ.* The process starts from the monomeric state at low concentration, having the intrinsic absorption spectrum of the substance, up to the high concentration showing the step-like behavior of the quantum confinement. However at all cases evidence for gradually growth of ordered structure formation expressed in the shoulder at the curve of the optical absorption was observed. The phenomenon occurs even at low concentrations probably due to existence of nuclei of the ordered structures.

Such ordered quantum confinement regions represent small crystalline area. The pronounced feature of these regions with crystalline ordered structure is that they grow due to self assembly process. In the self-assembly of flat, ring-shaped peptide subunits made up of alternating even number of D- and L-amino acid residues into extended tubular β-sheet-like structures, the intersubunit distance of the nanotube is about 4.8 Å with an 18 Å distance between the

striations. The internal diameter of these nanotubes is estimated to be about 7-13 Å , depending on the size of the subsequent.

Quantum well structures creates an electron confinement which produces changes in the optical and electrical properties of semiconductors structures. One of these changes is generation of photoluminescence. photoluminescence properties were observed for all types of the examined nanostructures. photoluminescence is a process in which a substance absorbs photons followed by re-radiation of photons. By absorbing the photons, the electrons are being excited to a higher energy state. The electrons in the excited states return to a lower energy state accompanied by the emission of photons.

FIG. 12 shows the photoluminescence and the excitation spectra of vapor deposition FF PNT along with the absorption spectrum. The excitation and photoluminescence spectra indicate that the FF PNT is being excited on the first absorption step, at 265 nm (4.7 eV) and it produces luminescent radiation mainly at the second step, at 296 nm (4.2 eV) but also toward the end of the third step, at about 450-470 nm (2.7-2.6 eV). There is a direct correlation between the excitation and photoluminescence spectra to the absorption spectrum indicating the reliability of the transition energies in the quantum confinement structures.

Notice that, unlike other semiconductor material possessing technologically embedded quantum confinement structures, the photoluminescence of the present embodiments is observed at room temperature. The transition energies are more intense and there are in the UV range of light.

The intense energy implies the involvement of a Frenkel exciton, which is an exciton of a small radius, and not a Mott-Wannier exciton which is typical to inorganic systems such as GaAs. The Frenkel exciton has a high typical binding energy on the order of 1.0 eV, hence the excitons tend to be much smaller, and of the same order as the unit cell (the electron and hole sit on the same cell). This is to contrary to the low binding energy of the Mott-Wannier exciton (around 0.1 eV) with a high radius, much larger then the lattice spacing.

Notice that the photoluminescence spectrum includes a second photoluminescence pick, in comparison to the intrinsic photoluminescence of the FF monomers. One of the optical properties in a quantum well structure relates to intraband (or inter-subband) transition energies. In quantum well structures the intraband transitions are noticeable due to the electronic states which are no longer in the form of the plane wave form. The intraband transitions in quantum well structures depend on the size of the confined region. It is postulated that the second pick of the photoluminescence is due to intraband transition since it has lower energy than the first pick and it appears only when the quantum confinement effect is observed.

FIGS. 13A-B show the excitation spectrum of Fmoc-FF and Fmoc-2-Nal hydrogels at various concentrations, respectively and FIG. 14 shows the excitation spectrum of Boc-FF and Fmoc-FF nanospheres at various concentrations, respectively. The excitation spectra show the process of crystallization of the quantum confined regions embedded in the structure. As shown, in low, sub-gel or below sphere formation, concentration the excitation spectrum is wide with multiple peaks due to lack of presence of the quantum confinement regions. In higher concentration, on the other hand, when gel or nanospheres form, the excitation spectrum becomes narrower up to extremely narrow width of about 5 nm, due to the incorporation of the vast majority of the monomers in the structure. The wide excitation spectrum of the sub-gel concentrations implies on the high density of the energy levels at the unoccupied energy levels and on the variety of the phononic interactions allowed at non-confines systems. At the gel concentration there is only one narrow excitation peak, this peak correspond to the exciton position. This finding is consistent with the narrow quantum confinement regions and well ordered and highly separated energy levels which do not allow electron-phonon interactions.

FIGS. 15A and 15B shows the photoluminescence of Fmoc-FF hydrogel at several concentrations and at two excitation wavelengths, at the intrinsic absorption peak, 270 nm (FIG, 15A), and in the narrow excitation peak of the quantum confined region, 310 nm (FIG. 15B). FIGS. 16A-B show the photoluminescence spectra of the Fmoc-FF nanospheres at several concentrations and excitation wavelengths.

The photoluminescence spectra of the different structures show the sensitivity of the spectrum to the excitation wavelength. This results demonstrate the quantization effect that is exhibited by the nanostructures. The results are consistent with the narrow peaks observed in FIGS. 13 and 14.

By following the photoluminescence spectrum during the process of the self assembly of the structures at different excitation wavelengths the present inventors successfully described this process.

Although the photoluminescence intensity varies according to the excitation wavelength, the location of the photoluminescence peak remains in the same wavelength. For Fmoc-FF nanospheres the photoluminescence peak is at 317 nm (3.9 eV), see FIGS. 16A and 16B, and for the hydrogel structure is at 325 nm (3.8 eV), see FIG. 15.

The size of the confined region can be estimated from the energy of the photoluminescence peak. The energy at two-dimensional quantum confinement structure can be described as:

$$E_{Z,n} = \frac{\hbar^2}{2m}\left(\frac{n\pi}{L_Z}\right)^2, \tag{1.1}$$

where $m$ is electron mass, $n$ is energy level, and $L_Z$ is the dimension of the confined region.

Assuming the transition of $n_1$=1 to $n_2$=2, $\Delta E$ is:

$$\Delta E = E_2 - E_1 = \frac{3\hbar^2\pi^2}{2mL^2}. \tag{1.2}$$

The calculated sizes of the confined regions in the structures according to the photoluminescence energy are: 5.2 Å for vapor deposition FF, 5.4 Å for Fmoc-FF hydrogel and nanospheres and 5.1 Å for Boc-FF nanospheres. Such small size quantum mechanically confined region are not possessed by traditional semiconductor technology.

***Example 2***

***Exemplified Blue Luminescence from Quantum Well Structures***

In this example, strong photoluminescence in blue and UV spectrum of exciton origin is described.

Optical properties of FF monomers and normally aligned PNT were stdied. Vapor deposition of PNT was onto quartz surfaces for detection of optical absorption up to 210 nm. The measurements were conducted using a Cary 5000 UV-Vis-NIR spectrophotometer (Varian, Inc. CA, USA) for the optical absorption, and FluoroMax®-3 spectrofluorometer (Horiba Jobin Yvon, NJ, USA) for the photoluminescence properties. A DMRB fluorescence microscope (Leica, Germany) was used for fluorescence imaging.

FIG. 17 shows the absorption spectrum of the PNT in comparison to FF monomers in aqueous solution. The spectrum of the monomers caused only by the aromatic rings of the phenylalanine residues, thus, it possess the same absorption peak (located at 257 nm) as the phenylalanine residue. However, the absorption spectrum of the aligned FF PNT demonstrates significantly different properties. The absorption spectrum of the FF PNT (solid line in FIG. 17) exhibits two distinguished steps located at 245-264 nm and 300-370 nm, compared to narrow absorption peak for FF monomers. The obtained step-like optical absorption behavior clearly indicates the existence of two-dimensional quantum confinement structures embedded in the FF PNT during self assembly process of deposition.

The PNT structures also generate strongly different photoluminescence compared to FF-monomer. FIGS. 18 shows the photoluminescence of the FF PNT and FF monomers under excitation at 260 nm (the excitation wavelength of the phenylalanine residue). The FF monomers possess a single peak ,located at 284 nm, that characterize the phenylalanine residue. The main peak of the FF PNT at this excitation wavelength is a sharp peak, located at 305 nm. A second peak was found in the range of 400-500 nm. The first peak is red-shifted by 11 nm (300 meV). Red-shifting of photoluminescence is already been observed at aromatic-based molecules upon aggregation [Pope, M.; Swenberg, C. E. Annu. Rev. Phys. Chem. 1984, 35, 613-655]. However, the red-shift in the present Example is higher in more than an order of magnitude from the examples that have been observed. The observed red-shift in the present example is ascribed to the crystallization process and formation of quantum confinement.

In order to investigate the photoluminescence nature of the PNT, the photoluminescence excitation (photoluminescence excitation) spectrum of the structures was measured at two emission wavelengths, at the first peak of 305 nm (dashed line, FIG. 19A) and at the second peak of 450 nm (solid line, FIG. 19A). As shown, the main origin of the 450 nm peak is located at 370 nm. At 260 nm the photoluminescence excitation intensity is about 15% from the intensity at 370 nm, hence the low intensity of the 450 nm photoluminescence peak shown in FIG. 18 (at excitation of 260 nm). The two photoluminescence excitation peaks are consistent with the red edges of the two absorption steps (solid line, FIG. 17).

FIG. 19B shows the photoluminescence intensity of the 450 nm peak under excitation at 370 nm (solid line) in comparison to the intensity of the 305 nm peak under excitation at 260 nm (dashed line). The 450 nm peak is about five times stronger then the 305 nm peak.

The variation in the optical properties of PNT in comparison to FF-monomer, particularly the step-like optical absorption indicates the creation of ordered quantum well structures. Such ordered structure indicates in turn anisotropic

self assembly growth. The longitudinal size of PNT along the Z-axis reaches a few micrometers. However, one of the transverse dimensions-width of the quantum well structure, $L_z$, is much smaller is in the nanoscale such that quantum confinement occurs. The calculation of the quantum well dimensions allows better understanding of the structure and dimensions of the elementary building blocks forming the PNT.

One of the distinguished features of the quantum confinement phenomenon is the strengthening of Coulomb interaction "electron-hole" and the creation of excitons, whose binding energy exceeds the corresponding value for the 3-D materials. The excitons are observed at the long-wavelength edge of the optical absorption spectra of the quantum well structure.

Excitons in molecular solids possess physical properties which are intermediate between the ones described by Wannier and Frenkel models. However, due to its simplicity the Wannier's exciton model is widely applied for estimation of exciton binding energy, $E_{exc}$, in molecular solids. This model gives reasonable values in the cases when $E_{exc}$ is sufficiently high and it exceeds the exciton binding energy in covalent semiconductors by one-two orders of magnitude. In the present example, the Wannier's exciton model, which does not take into account some specific features of Frenkel's exciton, was applied to organic quantum well structures, where excitons possess intermediate properties characterized by Wannier and Frenkel models. This technique allows the estimation of the parameters of localized excitons and can also be used for the determination of the quantum well's width based on experimentally measured optical properties.

The size $L_z$ in QW structure defines the exciton binding energy $\Delta E_{exc}$, its dimensionality and basic optical properties due to enhanced the exciton oscillator strength in a low-dimensional structures. $L_z$ may be estimated from the experimentally found value of $\Delta E_{exc}$. Theoretical calculations show that if $L_z$ is sufficiently smaller than the effective Bohr radius of exciton, $r_B$*, $L_Z << r_B$*, the three-dimensional exciton degenerates into two dimensions. The exciton binding energy depends on the width of the quantum well structure: for $L_Z << r_B$* it reaches its maximum value of $4Ry$* and it is reduced to $Ry$* for a wide quantum well $L_z >> r_B$*, here $Ry$* .is the effective Rydberg constant. For a quantum well of finite depth, the value of $\Delta E_{exc}$ may be found as $Ry^* < \Delta E_{exc} < 4Ry^*$. Increasing the exciton binding energy in confined quantum structures leads to pronounced exciton effects, observed in optical absorption and photoluminescence, not only at low but even at room and elevated temperatures (as demonstrated in FIG. 18). The value $r_B^*$ and $Ry$* are defined as:

$$r_B^* = \frac{\varepsilon_\infty \hbar^2}{\mu e_0^2} \quad . \qquad (2.1)$$

$$Ry^* = \frac{\mu e_0^4}{2\varepsilon_\infty^2 \hbar^2}, \qquad (2.2)$$

where $e_0$ is the elementary charge, $\mu$ is the reduced mass of the exciton $\mu = m_e m_h/(m_e+m_h)$, where $m_e$ and $m_h$ are the effective masses of electron and hole, respectively, and $\varepsilon_\infty$ is the high frequency dielectric permittivity. If the exciton binding energy is less than the energy of phonons, the value of $\varepsilon_\infty$ is replaced with the static dielectric permittivity $\varepsilon_0$.

For the sake of simplicity the exciton is considered in a potential well of infinite depth, when the carrier wave function does not penetrate into the surrounding medium.

The data on electron and hole potential wells, effective masses and dielectric permittivity for the exciton frequency ($\omega_{exc} = \Delta E_{exc} / .\hbar$), corresponding to exciton binding energy, is approximated using the model described in Bastard et al., Phys. Rev. B 1982, 26, (4), 1974-1979.

The experimental measurements show step-like optical absorption (FIG. 18) which might be understood as a transition between full and empty electronic states. Peaks localized at the "red" edge of these steps are related to direct optical excitation of the exciton. According to the data, the first absorption step is observed at $E_{step}^{(1)}$ = 4.11 *eV,* and the first exciton peak is found at the red edge of the first step, $E_{exc}^{(1)}$ = 3.13 *eV.* Thus, the value of the exciton binding energy is $\Delta E_{exc}^{(1)} = E_{step}^{(1)} - E_{exc}^{(1)}$ = 0.98 *eV,* which may be related to the states of the lowest subbands, with quantum numbers of transverse motion $n_e = n_h = 1$. The second exciton peak, at about 4.79 eV, which is clearly observed at the red edge of the second step, corresponds to $n_e = n_h = 2$. This step starts at approximately $E_{step}^{(2)}$ = 5.51 *eV.* It is noted that due to the quartz substance limitation, this energy region has a limitation to E = 5.9 *eV.*

The calculations of the electronic structure of excitons in GaAs quantum well, are presented by Bastard *et al. supra* in dimensionless units. The length is measured in units of Bohr's radius and the value of energy is measured in Rydberg constant $Ry$* in a dielectric medium. Assume that dependence of dimensionless exciton binding energy $\Delta E$ /

$Ry^*$ versus dimensionless quantum well length $L_Z / r_B^*$ is the same as it was obtained in Pope et al., Annu. Rev. Phys. Chem. 1984, 35, 613-655. However, the basic parameters are taken from the experimental results with PNT (FIG. 18). The value $\Delta E$ was found from optical absorption data for PNT $\Delta E_{exc}^{(1)} = 0.98$ *eV.* This value exceeds a maximum phonon energy, which allows using for $\varepsilon_\infty$, and its value for organic materials is about $\varepsilon_\infty = 4$.

Thus, there are two unknown variables: $L_Z$ and $\mu$, which will be extracted from two equations. A first equation can be written as:

$$\frac{L_Z}{r_B^*} = \frac{L_Z}{\varepsilon_\infty r_B} \frac{\mu}{m_0}, \qquad (2.3)$$

where $m_0 = 9.1 \times 10^{-28}$ g is the free-electron mass, $r_B = 0.529 \times 10^{-8}$ cm is Bohr's radius in a hydrogen atom. The dimensionless exciton binding energy is given by

$$\frac{\Delta E_{exc}^{(1)}}{Ry^*} = 1.152 \frac{m_0}{\mu}, \qquad (2.4)$$

where equations (2.3) and (2.4) are linked by the second curve in FIG. 17.

The second equation can be composed from the energy difference of the first and second steps in optical absorption spectrum (FIG. 18). The starting point of a step with quantum *numbers* $n = n_e = n_h$ is:

$$E_n = U + \frac{\pi^2 \hbar^2 n^2}{2\mu L_Z^2}, \qquad (2.5)$$

where U is the constant value which disappears in the final equation. The difference between energies of the second and first steps does not depend on U and cab be written as:

$$\Delta E_{12} = E_{step}^{(2)} - E_{step}^{(1)} = \frac{3\pi^2 \hbar^2}{2\mu L_Z^2} \qquad (2.6)$$

Equations (2.3) and (2.6) depend on $\mu$ and $L_Z$. Using the experimental values of the exciton binding energy, $\Delta E_{exc}^{(1)} = 0.98$ *eV and* $\Delta E_{12} = 1.64$ *eV,* the values $\mu = 0.86 m_0$ and $L_Z = 9$ Å are obtained.

The obtained data can be used to determine the exciton. The exciton has a high binding energy, $\Delta E_{exc}^{(1)} = 0.98$ *eV,* which significantly exceeds other known semiconductor materials due to much stronger confinement. The found width of the quantum well structure in PNT, $L_Z = 9$ Å , is slightly smaller than the calculated effective Bohr's diameter $2r_b^* = 13.8$ A, obtained for $\mu = 0.86 m_0$. The value of the exciton binding energy in the quantum well structure, $\Delta E_{exc} = 0.98$ eV, exceeds the binding energy of three-dimensional exciton $\Delta E_{3D} = Ry^* = 0.43$ *eV by* approximately two times, and it is two times less than the binding energy in two-dimensional space $\Delta E_{2D} = 4E_{3D} = 1.72$ eV. Therefore, the exciton can be related to an intermediate deformed state, occupying position between two-dimensional and three-dimensional excitons.

Excitons are effectively captured by shallow traps. Such exciton localization allows observing enormous growth of the oscillation strength, known as Rashba effect. It has been found that localization of the exciton in a quantum well structure that does not contain point defects, noticeably increases the oscillator strength of radiation transitions. Dramatic growth by 3-4 orders of magnitude of the oscillator strength may be observed in the case of the weak exciton localization at numerous shallow traps existing in quantum well structures. Such effect leads to fast exciton decay due to a strong growth of the radiative recombination rate and photoluminescence intensity. The same reason causes a sharp increase

optical absorption.

Another exciton-related effect is photoluminescence. The photoluminescence from PNT observed at room temperature demonstrates two peaks which may be ascribed to radiative decay of excitons, at 305 nm, located at the UV range, and at 450 nm, located at the blue range of the visible spectrum. The observed "red" shift (compared to the location of exciton optical adsorption peaks) can be related to Stocks effect. The full-width-at-half-maximum (FWHM) of the photoluminescence peaks are $\Delta\lambda_1$ = 33 nm and $\Delta\lambda_2$ =78 nm for the first and second photoluminescence peaks, respectively. The relatively wide second peak can be ascribed to electron-phonon interactions and/or influence of structural defects generated during the forming process of the PNT. The defects may lead to numerous overlapping optical transitions and provide significant widening of photoluminescence spectral bands.

FIG. 20 shows a fluorescence microscopy image of a patterned sample of FF PNT under excitation at 340-380 nm. FIG. 20 demonstrate blue photoluminescence from the PNT patterning in comparison to the dark purple reflection of the excitation beam, located in the center of the sample.

***Example 3***

***Exemplified Emissions from Quantum Dot Structures***

This example demonstrates peptide nanostructures exhibiting zero-dimensional quantum confinement structures, referred to as quantum dots.

Peptide nanospheres were formed by first dissolving Boc-FF building blocks in hexafluoro-2-propanol, followed by a dilution process to a desired concentration in 50% EtOH. The self-assembly process leads to the formation of peptide nanospheres structures with a wide diameters range of 40 nm to 1 $\mu$m. The use of $ddH_2O$ instead of EtOH resulted in aggregation of Boc-FF without peptide nanospheres.

FIGS. 21A and 21B show absorption spectra of peptide nanospheres (FIG. 21A) and unordered structures (FIG. 21B) for three concentrations. The optical absorption graphs for the peptide nanospheres, recorded for different Boc-FF concentrations (FIG. 21A) demonstrate a few separated peaks in the range of 240-280 nm. The position of the individual peaks and the spectral structure of the optical absorption curves do not change with the peptide concentration, while the intensity of the peaks increases. The absorption spectrum of unordered structures (FIG. 21B) has no unique features in comparison to the spectrum of the peptide nanospheres.

The recorded spectra FIG. 21A of the optical absorptions of peptide nanospheres have spike-like spectral structures indicating the formation of quantum dot structures in a peptide nanosphere.

Quantum confinement structures are characterized by enhancement of exciton effects, providing increase of its binding energy and oscillator strength, which facilitates an exciton luminescence at room temperature or higher temperatures.

FIGS. 22A and 22B shows photoluminescence excitation (PLE) spectra of the peptide nanospheres (FIG. 22A) and the unordered structures (FIG. 22B) at several concentrations. The emission wavelength is 282 nm. At low concentrations, when most of the Boc-FF building blocks have not been self-assembled, the photoluminescence excitation spectrum is wide with a multi-peak shape. As the concentration increases and more building blocks self-assemble into the peptide nanospheres, both absorption and photoluminescence excitation spectra are identical and their peaks are located at 265 nm (4.68 eV), 259 nm (4.79 eV), 253 nm (4.90 eV), and 248 nm (5.0 eV). The energy interval between two neighboring peaks, both for absorption and for photoluminescence excitation, is the same and is approximately 0.10-0.11 eV. The photoluminescence excitation peak that relates to the excitation at 270 nm has the highest intensity. The intensity of the other peaks gradually and monotonically decreases with their transition from the main 265 nm peak.

The main 270 peak is well defined and narrow (full width at half maximum of about 7 nm), and indicates the creation of exciton. The forming of the narrow peak is direct evidence of the crystalline structure formed in the peptide nanospheres. Moreover, the low line-shape broadening of the excitonic transitions in the spectra at room temperature demonstrates the high nanocrystal quality. On the other hand, the photoluminescence excitation spectrum of the unordered structures (FIG. 22B) does not show the forming of the exciton peak. At all the concentrations, the photoluminescence excitation spectrum is wide.

The optical properties of quantum dot depend on their size. Strong spatial exciton confinement in quantum dot results in a pronounced difference of exciton optical properties from those observed in an infinite crystal. The electronic structure of the exciton is defined by the relation of the quantum dot radius R to a Bohr radius $r_B$ of the exciton (see Equation 2.1 in Example 2). A high-frequency dielectric constant in organic materials does not exceed more then a few units. In this quantum dot structure, the exciton radius $r_B$ is about a few angstroms, while a typical value of $R$ is higher by about an order of magnitude, that is $r_B << R$. Such a relation provides the conditions for a weak confinement when exciton motion may be considered as an almost free motion inside the quantum dot.

Quantum dot systems can be described using a model of infinitely deep spherical potential wells. The spectral position of the main exciton absorption line at a weak confinement is given by

$$\hbar\omega = E_g - E_{ex} + \frac{\hbar^2\pi^2}{2MR^2}, \qquad (3.1)$$

where Eg is the band gap of the quantum dot material, $M = m_e + m_h$ is the translation mass of the exciton, and $E_{ex}$ is the binding energy of the exciton in an infinite crystal, given by

$$E_{ex} = \frac{\mu e^4}{2\hbar^2\varepsilon_\infty^2}. \qquad (3.2)$$

The experimental data (FIGS. 21A and 22A) the photoluminescence excitation peak that related to the excitation at 265 nm has the highest intensity. The intensity of the other photoluminescence excitation peaks gradually decreases, where the larger the energy interval between the fundamental absorption peaks the less its intensity. Such an absorption and photoluminescence excitation behavior is typical for local centers where the excited electron interacts with lattice vibrations. Therefore the observed spectrum (FIG. 21A) can be the effect of the phononless exciton absorption line at 265nm and its phonon replicas at 259 nm, 253 nm, and 248 nm. The energy interval between the resulting maxima is equal to the phonon energy $\hbar\omega_{ph}$ = 0.10 - 0.11$eV$, which actively interacts with the excited exciton.

FIG. 21A shows that the continuous optical absorption band starts from $\lambda \leq \lambda_{ion}$ = 242 nm ($\hbar\omega_{ion}$ = 5.12eV), which may be interpreted as the breaking of the binding exciton state. The value of $\hbar\omega_{ion}$ corresponds to the energy gap of the quantum dot, which is consistent with the value of the transport gap of approximately 5.1 eV found for molecular benzenethiol crystals [I. J. Lalov and I. Zhelyazkov, Phys. Rev. B 75 (2007), C. D. Zangmeister, S. W. Robey, R. D. van Zee, Y. Yao, and J. M. Tour, J. Phys. Chem. B 108, 16187 (2004)]. The electronic structure of this aromatic crystal is close to the studied material. The difference between $\hbar\omega_{ion}$ and the phononless band near $\hbar\omega_g^0$ is 0.44 eV. This energy represents the exciton binding energy, $E_{ex}^{QD}$, of the Boc-FF quantum dot, which is higher than that in GaAs by approximately two orders of magnitude. Such tightly bound excitons are responsible for the pronounced photoluminescence observed at room temperature.

From equations (3.1) and (3.2) it follows that

$$E_{ex}^{QD} = \hbar\omega_{ion} - \hbar\omega_g = E_{ex} - \frac{\hbar^2\pi^2}{2MR^2}, \qquad (3.3)$$

and from equations (3.2) and (3.3) the radius of the quantum dot can be estimated as

$$R = \pi r_B^0 \sqrt{\frac{\frac{m_0}{M}}{\frac{\mu}{m_0\varepsilon_\infty^2} - \frac{E_{ex}^{QD}}{Ry}}}, \qquad (3.4)$$

where $r_B^0 = \hbar^2 / m_0 e^2 = 0.529$ Å is the Bohr radius of the hydrogen atom, $m_0$ is the free electron mass, and $Ry = m_0e^4 / 2\hbar^2$ = 13.56$eV$ is the Rydberg constant.

The size of the quantum dot is estimated from the optical measurements and the related molecular aromatic benzene crystal, which has an identical structure of the aromatic ring. The refractive index of the benzene crystal is n = 1.501, hence $\varepsilon_\infty = n^2$ = 2.253. In accordance to the electronic model of 1,4-diiodobenzene crystal, the effective mass of electrons and holes is almost equal and close to 0.5$m_0$. Then, for $\mu = 1/2m_e = 0.25m_0$ and for $M = m_0$ equation (3.4) estimates the value of the quantum dot, R which is approximately 1.3 nm.

Quantum dot are considered as spherical nanocrystalline particles embedded into a material matrix of another origin. In the present example, the calculated size of the quantum dot suggests the presence of small quantum dot

crystalline regions embedded along the peptide nanospheres. It is postulated that the crystalline regions comprised from the aromatic rings of the phenylalanine residues are due to the similarity of the electronic structures of benzene-related materials. In this case the boundaries for the confined regions are the Boc group and the peptide backbone.

FIGS. 23A-D shows photoluminescence spectrum of the peptide nanospheres (FIGS. 23A and 23B) and unordered structures (FIGS. 23C and 23D) at concentrations of 4 mg/ml (red solid line) and 1 mg/ml (black dashed line) at excitation wavelengths of 270 nm (FIGS. 23A and 23C) and 255 nm (FIGS. 23B and 23D). The photoluminescence excitation spectrum and the Stokes shift (15 nm) are shown in FIG. 23A.

At common chemical solutions (with no peptide nanospheres), the photoluminescence of a sample is proportional to the concentration, regardless of the excitation wavelength. This is demonstrated FIGS. 23C and 23D. On the other hand, under the condition where the peptide buildings blocks are self-assembled into peptide nanospheres (FIGS. 23A and 23B) the tendency is different. At low concentration solution the conditions are below the threshold for effective peptide nanospheres forming, thus such a solution contains mainly unordered building blocks rather than peptide nanospheres. At higher concentrations almost all of the building blocks have been self assembled into peptide nanospheres.

When the solutions are excited at the wavelength in the range of exciton wavelength (270 nm), the concentrated solution has a greater intensity than the low concentrated sample due to higher concentration of peptide nanospheres (FIG. 23A). However, when the same solutions are excited at 255 nm, the result is reversed (FIG. 23B). The low concentration solution, containing mainly unordered building blocks, has a stronger photoluminescence than the high concentration solution, with solely peptide nanospheres. This is due to the narrow excitation peak of the peptide nanospheres.

### *Example 4*

### *Exemplified Quantum confinement in Amyloid fibrils*

The present example describes experiment performed in accordance with some embodiments of the present invention to demonstrate quantum confinement in amyloid fibrils. FIG. 24A is an AFM image of insulin fibrils, and FIG. 24B shows a cross-section of two insulin fibrils along the line marked by block arrow in FIG. 24A. The diameter of the fibrils is about 6 nm.

FIG. 25A shows absorption spectrum of 0.5 mg/ml insulin, immediately (within a few minutes) following the preparation of the solution (designated "0 hours" in FIG. 25A) and 2 hours from the preparation of the solution; and FIG. 25B shows photoluminescence excitation spectrum of insulin, as measured immediately (within a few minutes) following the preparation of the solution from the preparation of the solution. The excitation spectrum was measured at emission wavelength of 305 nm.

As shown the insulin fibrils exhibit a step-like optical absorption which indicate the formation of quantum well structures. Note that although the photoluminescence excitation spectrum was taken at 0 hours there are significant changes between the different concentrations of the insulin building blocks. At low concentrations, the spectrum is composed from a main peak located at about 276 nm. This corresponds to insulin excitation. At this low concentration there is another peak, of lower intensity, located at 230-235 nm (depending on the concentration). This peak was discovered by the present inventors.

As the concentration increase, the shape of the photoluminescence excitation spectrum gradually changes. The monomeric peak at 276 nm disappears and two new peaks appear. These peaks were also discovered by the present inventors. At concentration of 4 mg/ml, the position of the main new peak is at about 287 nm, and is width is about 7 nm FWHM. This peak indicates formation of exciton, as described in Examples 1-3 above. The second new peak is located at about 260-270 nm (depending on the concentration).

The aggregation of insulin and other amyloid fibrils is a rather slow process that includes a long lag phase of 2-3 hours, at laboratory conditions. At this phase, the insulin building blocks are soluble within the solution. Following this phase the insulin building blocks aggregate and become insoluble. The result presented in the present example demonstrates that quantum confinement can be determined already while the insulin building blocks are soluble. Such effect indicates occurrence of an ultra-fast crystallization process in the solution.

## Claims

**1.** A light emitting system, comprising a plurality of peptide nanostructures forming organic sub-nanometric crystalline structures which exhibit quantum confinement, and means configured to excite said sub-nanometric crystalline structures to emit light, wherein said means is selected from the group consisting of;
a pair of electrodes configured to excite said sub-nanometric crystalline structures by injecting holes and electrons to said peptide nanostructures, thereby to emit light via injection luminescence,

a heat source configured to excite said sub-nanometric crystalline structures by heating said peptide nanostructures, thereby to emit light via thermoluminescence, and
means which comprises or is connectable to a voltage source configured to excite said sub-nanometric crystalline structures by applying voltage to said peptide nanostructures, thereby to emit light via electroluminescence.

**2.** A method of emitting light comprising exciting a plurality of peptide nanostructures forming organic sub-nanometric crystalline structure which exhibits quantum confinement, so as to emit light, wherein said exciting is selected from the group consisting of using a voltage source for generating electric field for exciting said sub-nanometric crystalline structure to emit light via electroluminescence, using a pair of electrodes for injecting holes and electrons to said sub-nanometric crystalline structure to emit light via injection luminescence, and heating said sub-nanometric crystalline structure to emit light via thermoluminescence.

**3.** A method of predicting formation of an amyloid plaque in a peptide sample, comprising determining presence or absence of quantum confinement in the sample,
wherein presence of quantum confinement in the sample indicates that formation of an amyloid plaque is likely to occur,
whereas absence of quantum confinement in the sample indicates that formation of an amyloid plaque is not likely to occur.

**4.** The method of claim 3, wherein an amount of soluble peptides in said peptide sample is at least 2 times higher than an amount of insoluble peptides in said solution.

**5.** The method of claim 3, wherein said peptide sample is substantially devoid of insoluble peptides.

**6.** The method according to any of claims 3-5, wherein said determination is by measuring at least one spectrum selected from the group consisting of an optical absorption spectrum and a photoluminescence excitation spectrum.

**7.** The method according to any of claims 3-6, wherein the sample contains insulin, wherein said determination is by measuring a photoluminescence excitation spectrum, and wherein said quantum confinement is manifested as a sufficiently narrow peak between a wavelength of 280 nm and a wavelength of 295 nm.

**Patentansprüche**

**1.** Lichtemittierendes System, das eine Mehrzahl von Peptidnanostrukturen, die organische subnanometrische kristalline Strukturen bilden, die ein Quanten-Confinement aufweisen, und ein Mittel umfasst, das so konfiguriert ist, dass es die subnanometrischen kristallinen Strukturen anregt, so dass Licht emittiert wird, wobei das Mittel aus der Gruppe ausgewählt ist, bestehend aus:

einem Paar von Elektroden, die so konfiguriert sind, dass sie die subnanometrischen kristallinen Strukturen durch Injizieren von Löchern und Elektronen in die Peptidnanostrukturen anregen, um dadurch Licht mittels Injektionslumineszenz zu emittieren,
einer Wärmequelle, die so konfiguriert ist, dass sie die subnanometrischen kristallinen Strukturen durch Erhitzen der Peptidnanostrukturen anregt, um dadurch Licht mittels Thermolumineszenz zu emittieren, und
einem Mittel, das eine Spannungsquelle umfasst oder mit dieser verbindbar ist, das so konfiguriert ist, dass es die subnanometrischen kristallinen Strukturen anregt, indem es eine Spannung an die Peptidnanostrukturen anlegt, um dadurch Licht mittels Elektrolumineszenz zu emittieren.

**2.** Verfahren zum Emittieren von Licht, das das Anregen einer Mehrzahl von Peptidnanostrukturen umfasst, die eine organische subnanometrische kristalline Struktur bilden, die ein Quanten-Confinement aufweisen, um Licht zu emittieren, wobei das Anregen aus der Gruppe ausgewählt ist, bestehend aus Verwenden einer Spannungsquelle zum Erzeugen eines elektrischen Feldes zum Anregen der subnanometrischen kristallinen Struktur, um Licht mittels Elektrolumineszenz zu emittieren, das Verwenden eines Paares von Elektroden zum Injizieren von Löchern und Elektronen in die subnanometrische kristalline Struktur, um Licht mittels Injektionslumineszenz zu emittieren, und das Erhitzen der subnanometrischen kristallinen Struktur umfasst, um Licht mittels Thermolumineszenz zu emittieren.

**3.** Verfahren zum Prognostizieren einer Bildung von amyloidem Plaque in einer Peptidprobe, das das Ermitteln des

Vorhandenseins oder Nicht-Vorhandenseins eines Quanten-Confinements in der Probe umfasst,
wobei das Vorhandensein eines Quanten-Confmements in der Probe anzeigt, dass das Auftreten einer Bildung von amyloidem Plaque wahrscheinlich ist,
wobei das Nicht-Vorhandensein eines Quanten-Confinements in der Probe anzeigt, dass das Auftreten einer Bildung von amyloidem Plaque unwahrscheinlich ist.

**4.** Verfahren nach Anspruch 3, wobei eine Menge von löslichen Peptiden in der Peptidprobe zumindest zweimal größer als eine Menge von unlöslichen Peptiden in der Lösung ist.

**5.** Verfahren nach Anspruch 3, wobei die Peptidprobe im Wesentlichen frei von unlöslichen Peptiden ist.

**6.** Verfahren nach einem der Ansprüche 3 bis 5, wobei das Ermitteln durch Messen zumindest eines Spektrums erfolgt, das aus der Gruppe ausgewählt ist, bestehend aus einem optischen Absorptionsspektrum und einem Photolumineszenzanregungsspektrum.

**7.** Verfahren nach einem der Ansprüche 3 bis 6, wobei die Probe Insulin enthält, wobei das Ermitteln durch Messen eines Photolumineszenzanregungsspektrums erfolgt, und wobei sich das Quanten-Confinement als ausreichend enge Spitze zwischen einer Wellenlänge von 280 nm und einer Wellenlänge von 295 nm manifestiert.

## Revendications

**1.** Système d'émission de lumière, comprenant une pluralité de nanostructures peptidiques formant des structures cristallines sub-nanométriques organiques qui présentent un confinement quantique, et des moyens configurés pour exciter lesdites structures cristallines sub-nanométriques pour émettre de la lumière, lesdits moyens étant choisis dans le groupe constitué de :

une paire d'électrodes configurées pour exciter lesdites structures cristallines sub-nanométriques par injection de trous et d'électrons dans lesdites nanostructures peptidiques, de manière à émettre de la lumière via luminescence par injection,
une source de chaleur configurée pour exciter lesdites structures cristallines sub-nanométriques par chauffages desdites nanostructures peptidiques, de manière à émettre de la lumière via thermoluminescence, et
les moyens qui comprennent ou peuvent être raccordés à une source de tension configurés pour exciter lesdites structures cristallines sub-nanométriques par application de tension auxdites nanostructures peptidiques, de manière à émettre de la lumière via électroluminescence.

**2.** Procédé d'émission de lumière comprenant l'excitation d'une pluralité de nanostructures peptidiques formant une structure cristalline sub-nanométrique organique qui présente un confinement quantique; de manière à émettre de la lumière, dans lequel ladite excitation est choisie dans le groupe constitué de l'utilisation d'une source de tension pour générer un champ électrique pour exciter ladite structure cristalline sub-nanométrique de manière à émettre de la lumière via électroluminescence, l'utilisation d'une paire d'électrodes pour injecter des trous et des électrons dans ladite structure cristalline sub-nanométrique de manière à émettre de la lumière via luminescence par injection, et le chauffage de ladite structure cristalline sub-nanométrique de manière à émettre de la lumière via thermoluminescence.

**3.** Procédé de prédiction de la formation d'une plaque amyloïde dans un échantillon de peptide, comprenant la détermination de la présence ou l'absence de confinement quantique dans l'échantillon,
dans lequel la présence de confinement quantique dans l'échantillon indique que la formation d'une plaque amyloïde est susceptible de se produire,
tandis que l'absence de confinement quantique dans l'échantillon indique que la formation d'une plaque amyloïde n'est pas susceptible de se produire.

**4.** Procédé de la revendication 3, dans lequel une quantité de peptides solubles dans ledit échantillon de peptide est au moins 2 fois plus élevée qu'une quantité de peptides solubles dans ladite solution.

**5.** Procédé de la revendication 3, dans lequel ledit échantillon de peptide est sensiblement exempt de peptides insolubles.

**6.** Procédé selon l'une quelconque des revendications 3 à 5, dans lequel la détermination est effectuée par mesure d'au moins un spectre choisi dans le groupe constitué d'un spectre d'absorption optique et d'un spectre d'excitation de photoluminescence.

**7.** Procédé selon l'une quelconque des revendications 3 à 6, dans lequel l'échantillon contient de l'insuline, ladite détermination étant effectuée par mesure d'un spectre d'excitation de photoluminescence, et ledit confinement quantique se manifestant par un pic suffisamment étroit entre une longueur d'onde de 280 nm et une longueur d'onde de 295 nm.

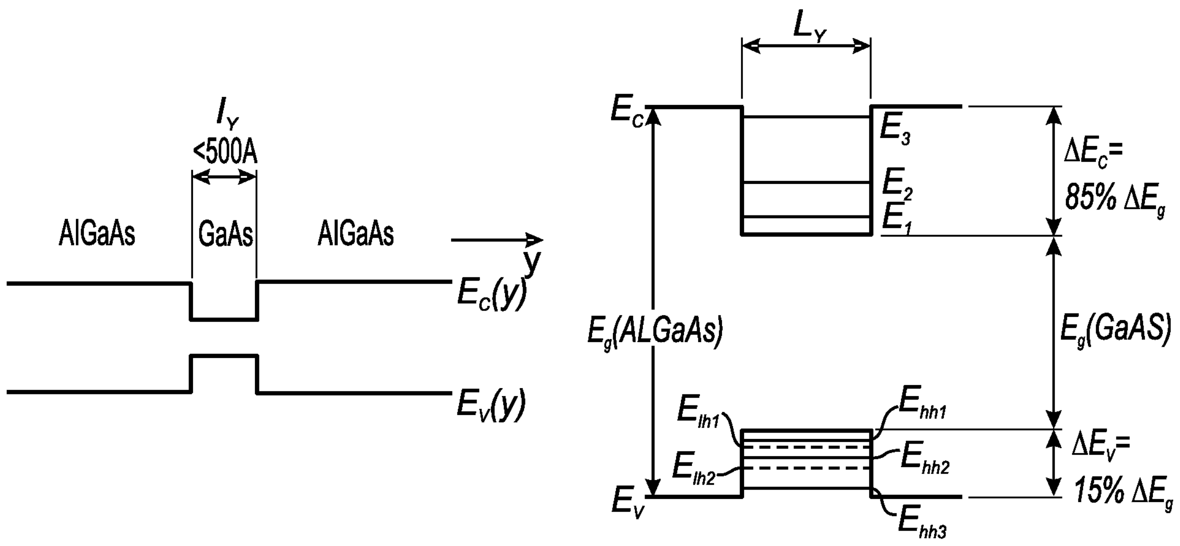


FIG. 1

FIG. 2A

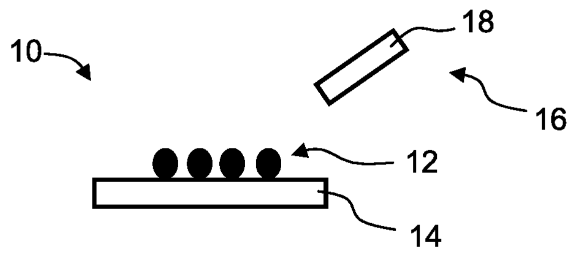


FIG. 2B

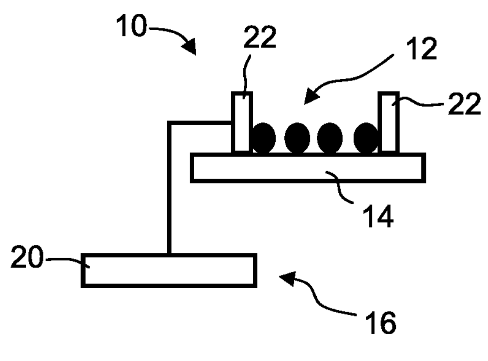


FIG. 2C

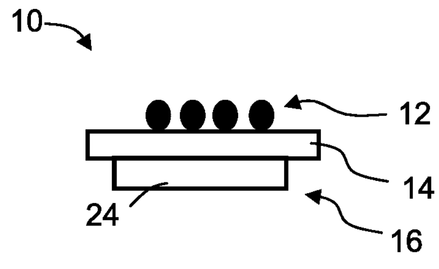

FIG. 3A

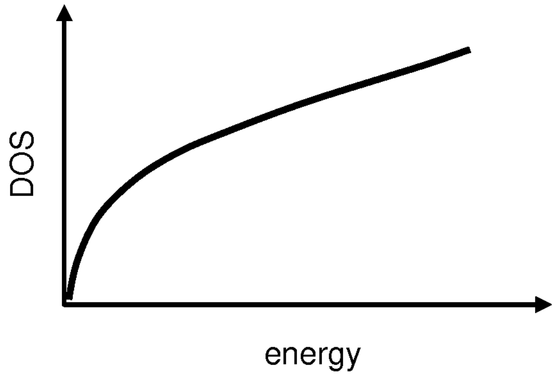


FIG. 3B

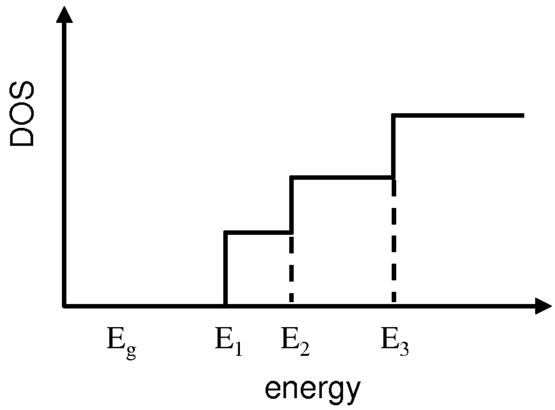


FIG. 3C

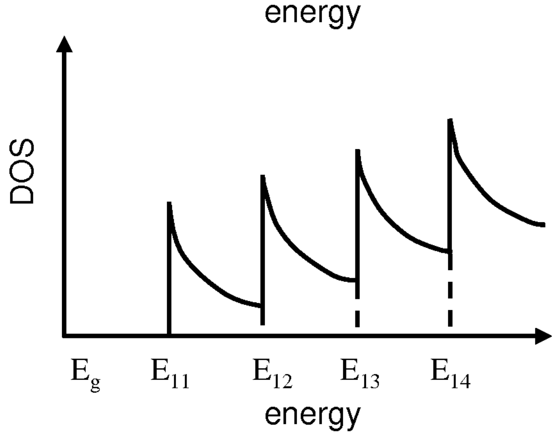


FIG. 3D

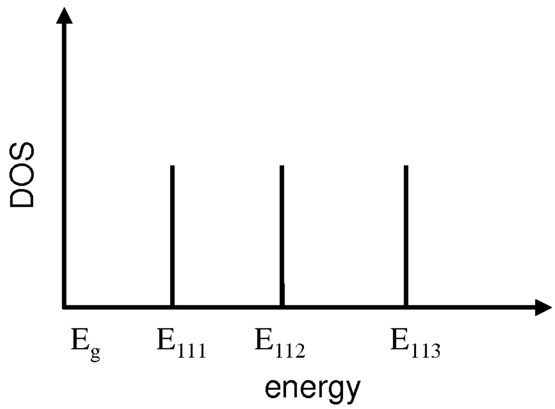

FIG. 4

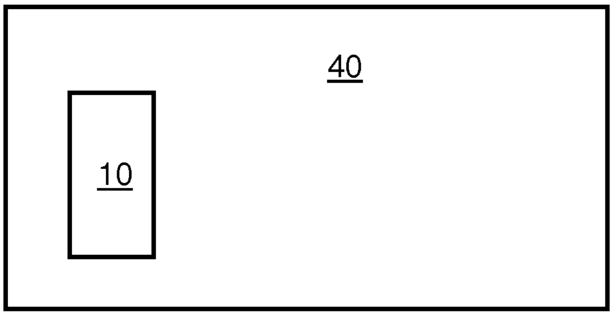


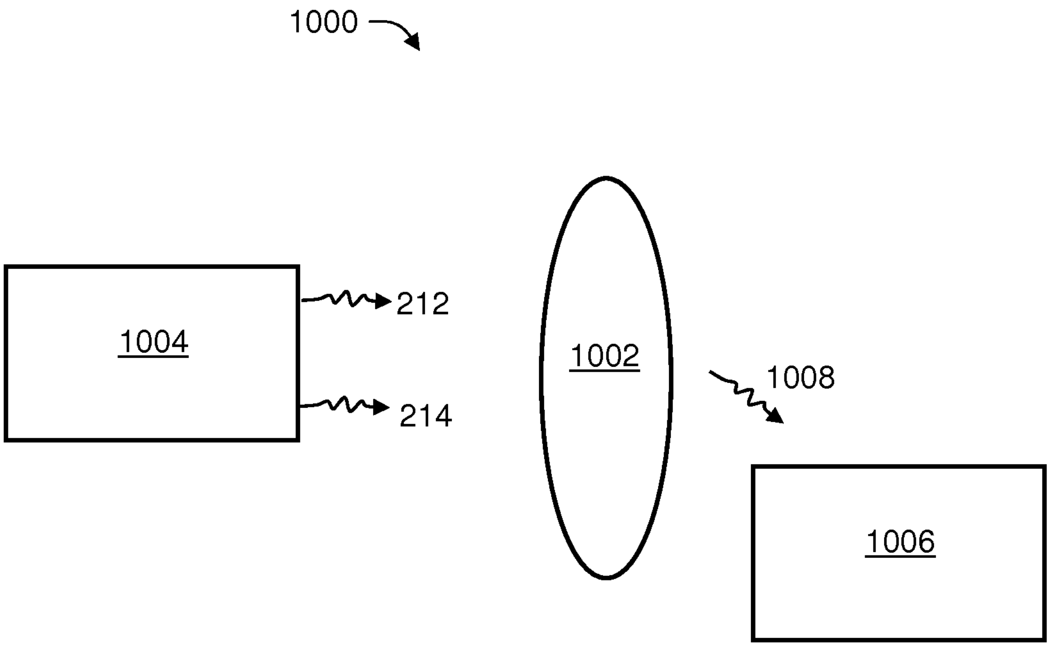


FIG. 5A

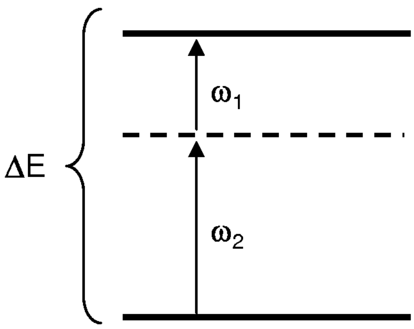


FIG. 5B

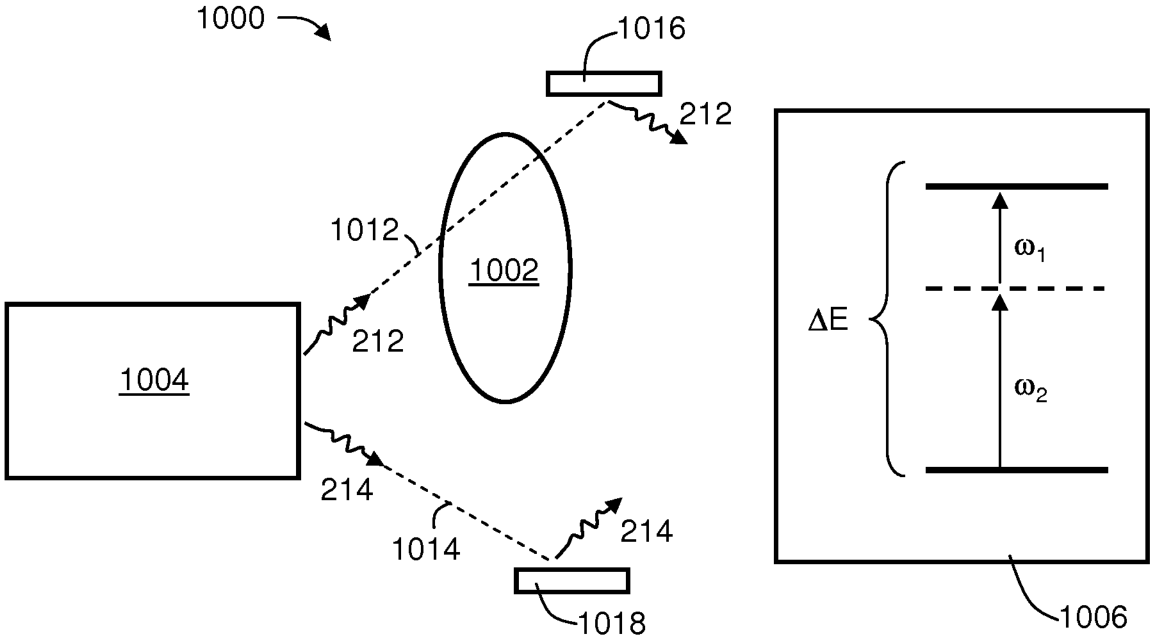
1000
1016
212
1012
1002
1004
212
214
1014
214
1018
ΔE
ω1
ω2
1006

FIG. 5C

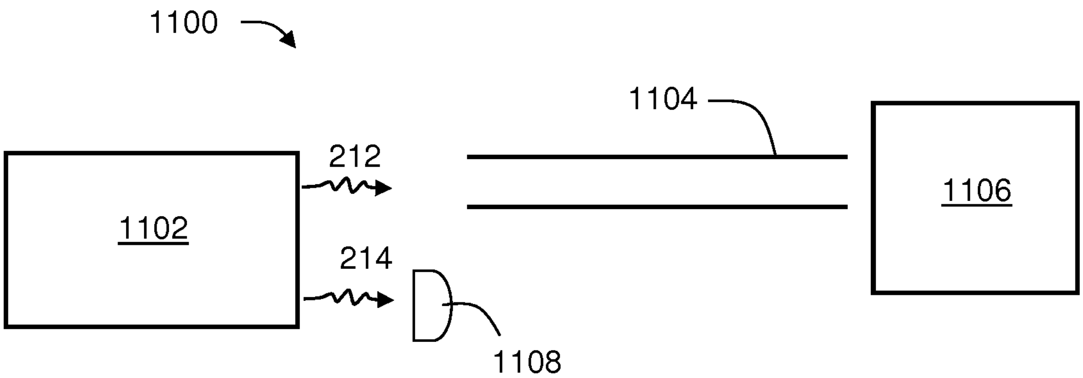
1100
1104
1102
212
214
1106
1108

FIG. 6

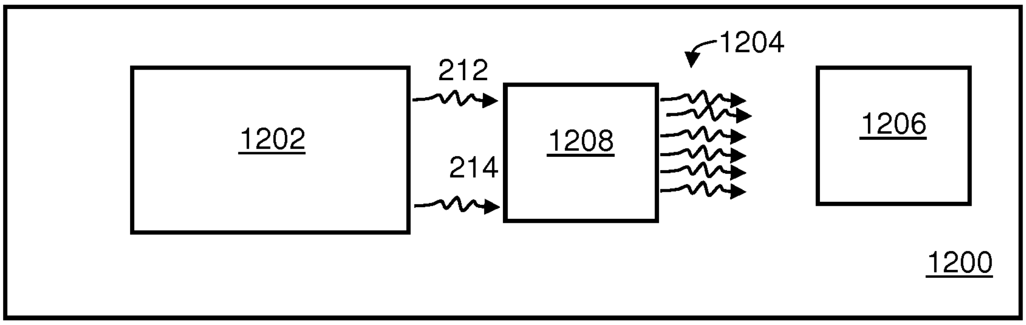
212
214
1202
1208
1204
1206
1200

FIG. 7

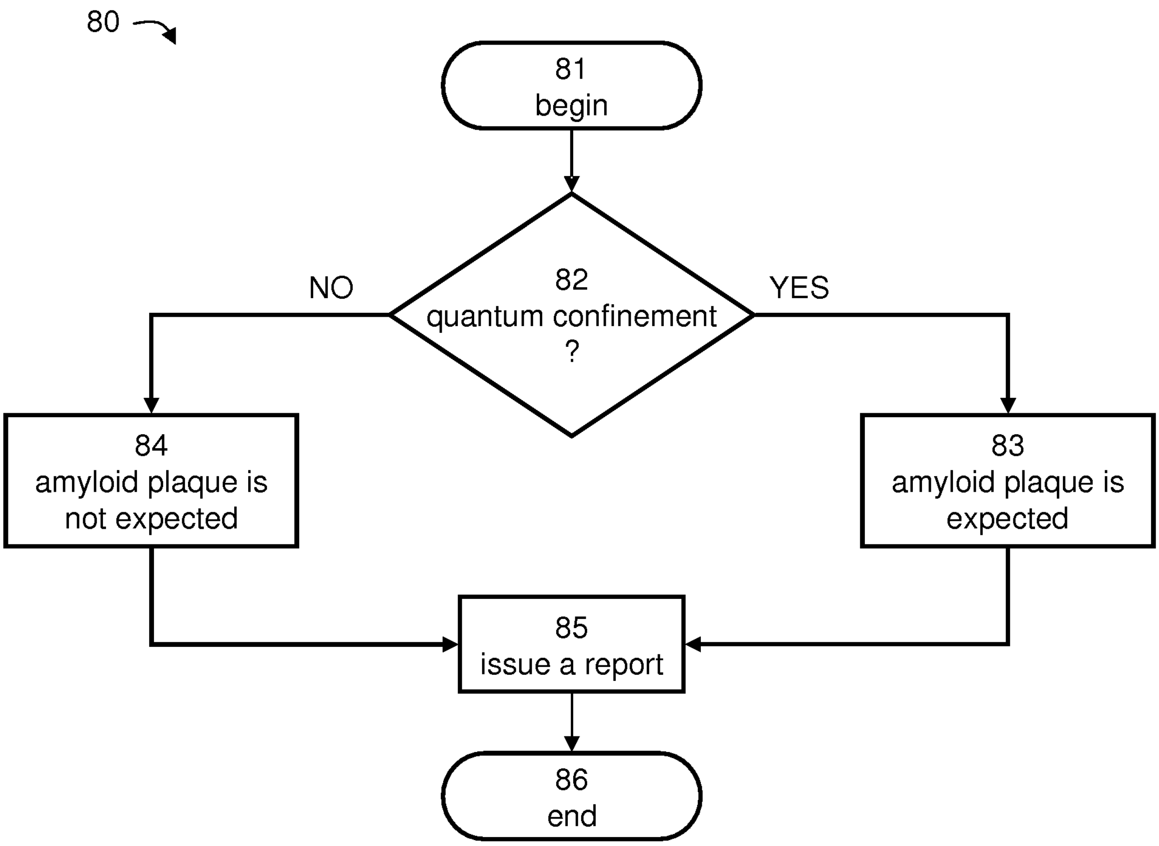
80
81
begin
82
quantum confinement
?
NO
YES
84
amyloid plaque is
not expected
83
amyloid plaque is
expected
85
issue a report
86
end

FIG. 8

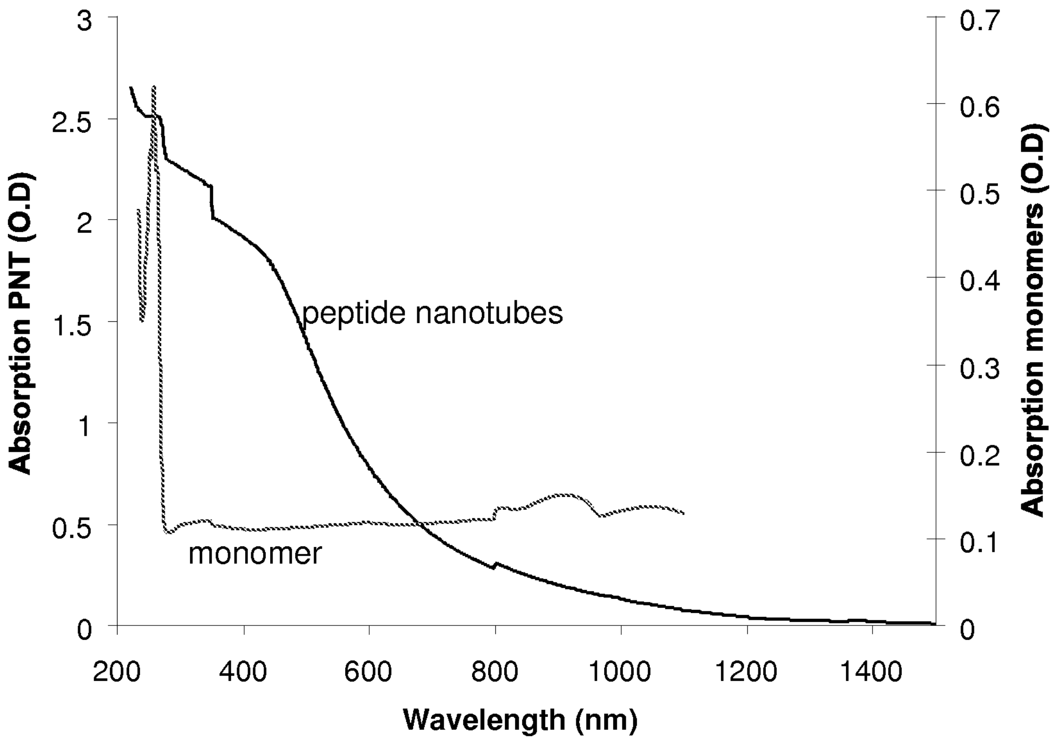

3
2.5
2
1.5
1
0.5
0
Absorption PNT (O.D)
0.7
0.6
0.5
0.4
0.3
0.2
0.1
0
Absorption monomers (O.D)
peptide nanotubes
monomer
200
400
600
800
1000
1200
1400
Wavelength (nm)


FIG. 9

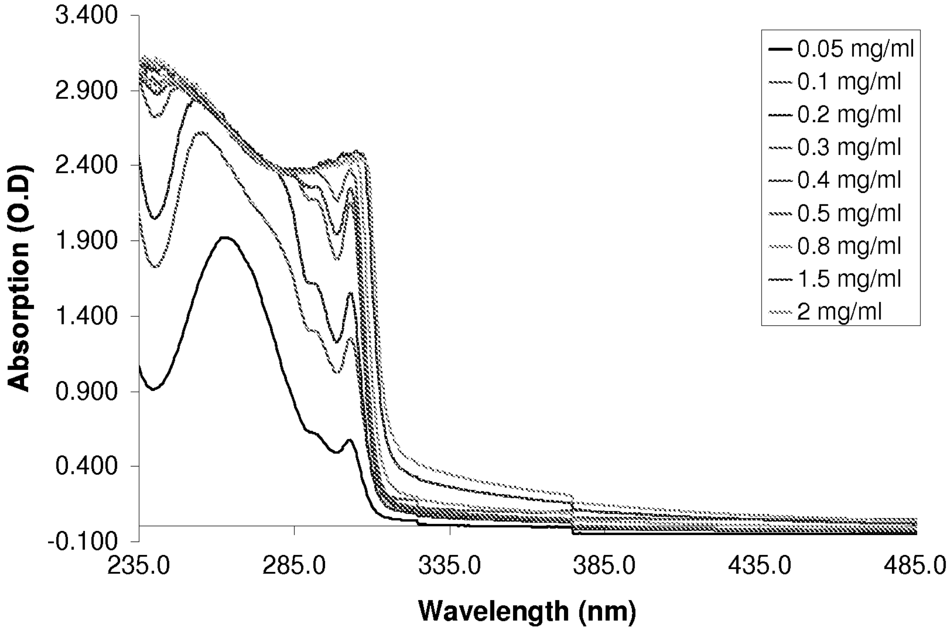


FIG. 10A

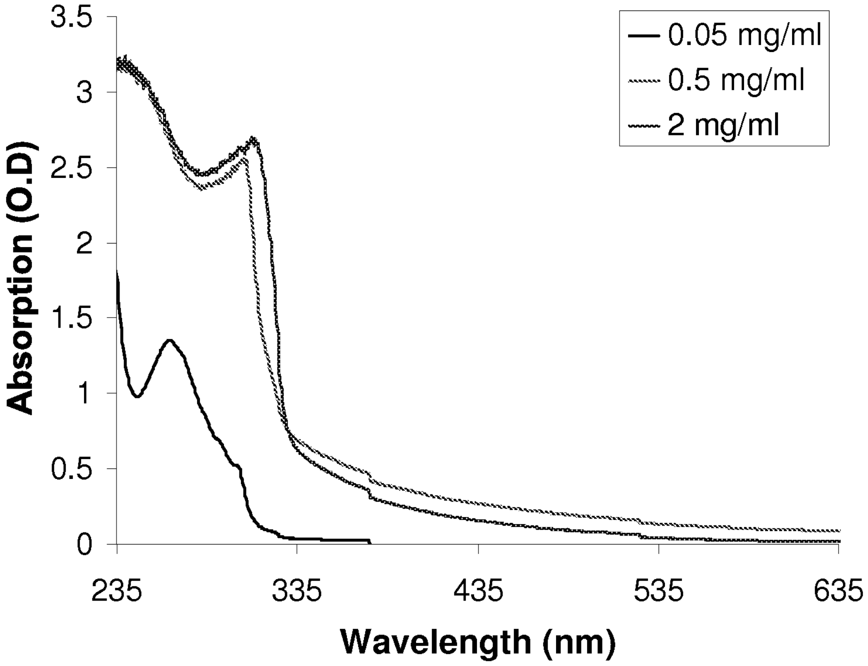


FIG. 10B

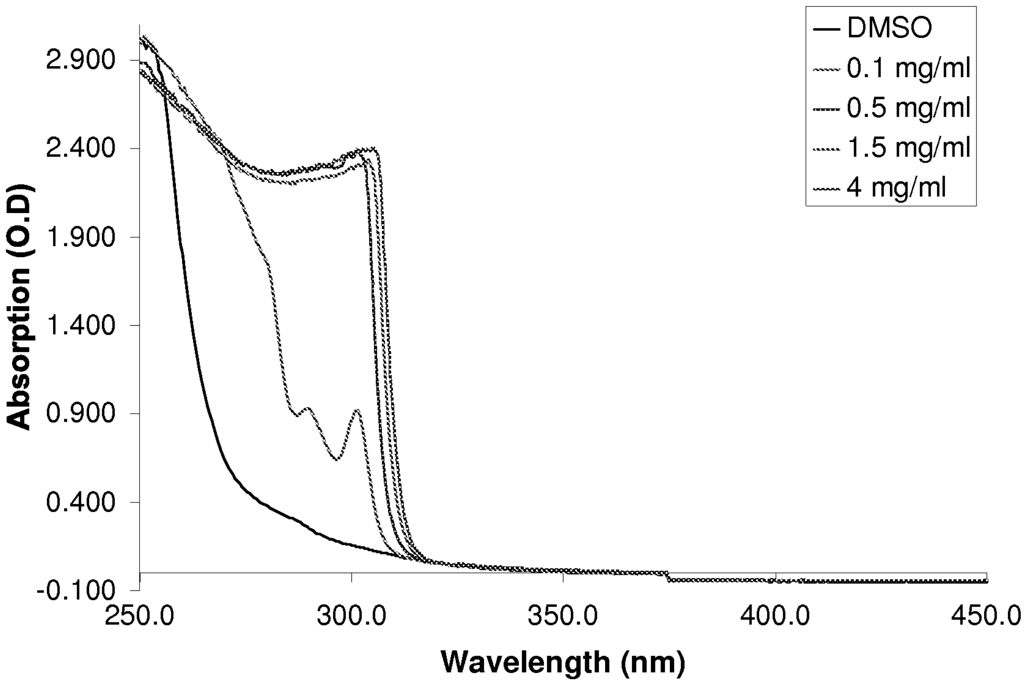
DMSO
0.1 mg/ml
0.5 mg/ml
1.5 mg/ml
4 mg/ml
Absorption (O.D)
2.900
2.400
1.900
1.400
0.900
0.400
-0.100
250.0
300.0
350.0
400.0
450.0
Wavelength (nm)

FIG. 11

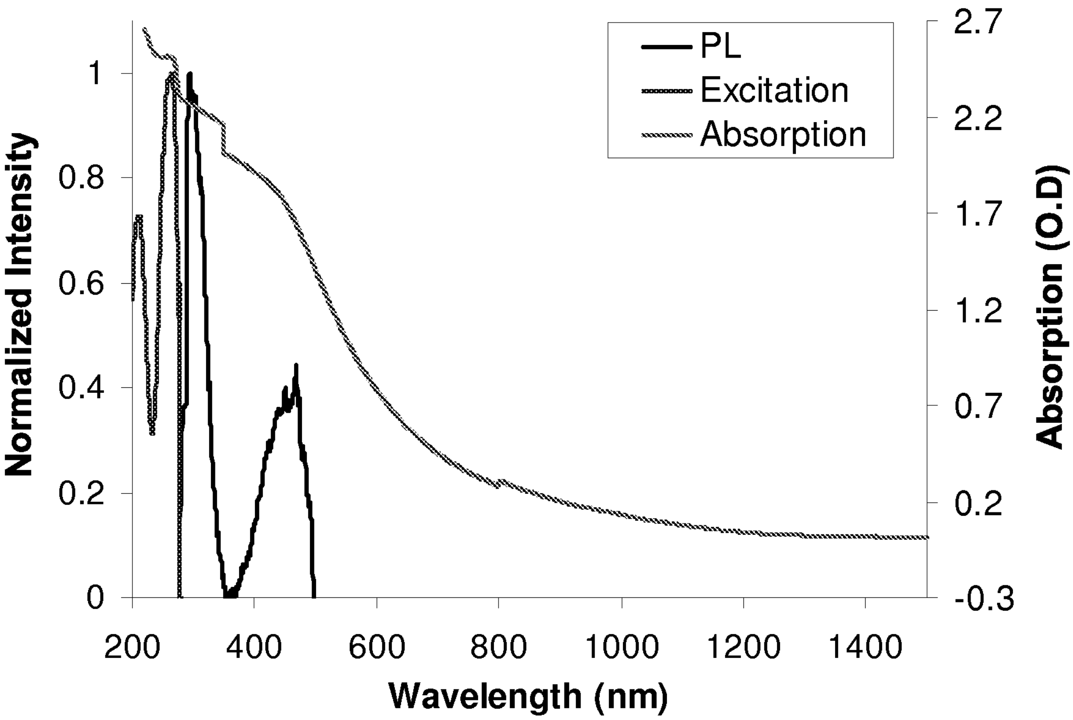
PL
Excitation
Absorption
Normalized Intensity
1
0.8
0.6
0.4
0.2
0
2.7
2.2
1.7
1.2
0.7
0.2
-0.3
Absorption (O.D)
200
400
600
800
1000
1200
1400
Wavelength (nm)

FIG. 12

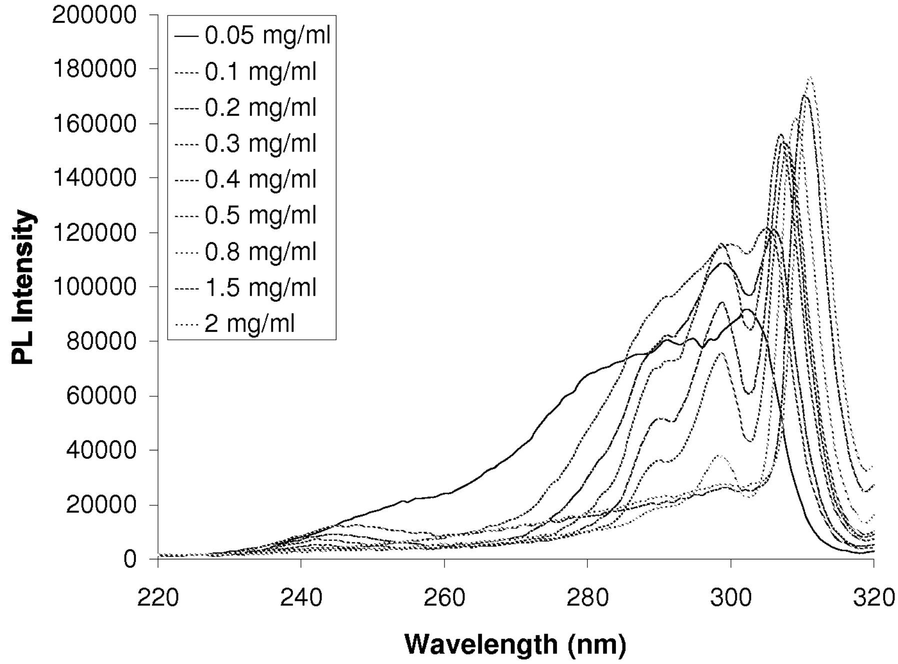


FIG. 13A

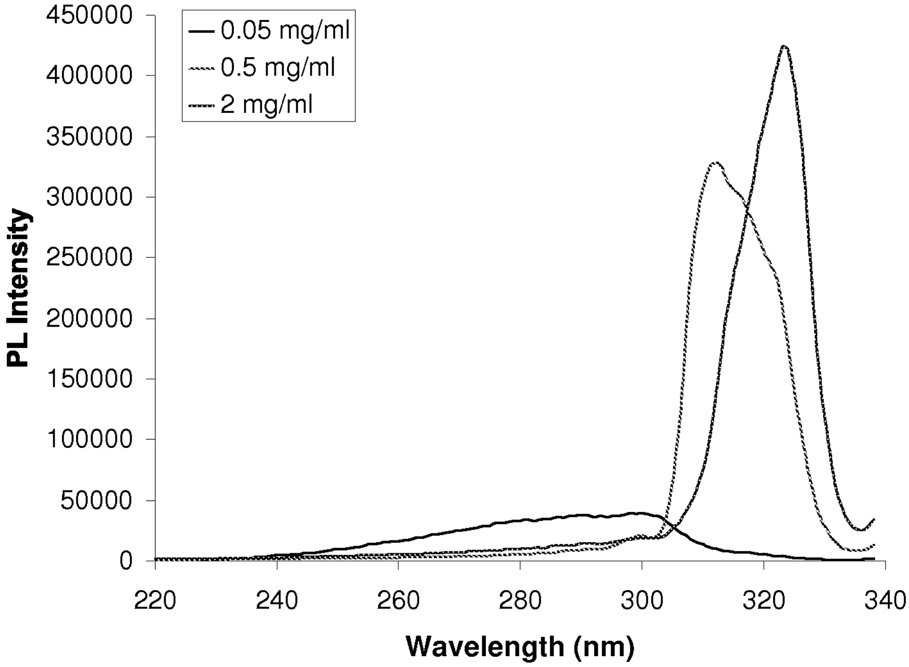


FIG. 13B

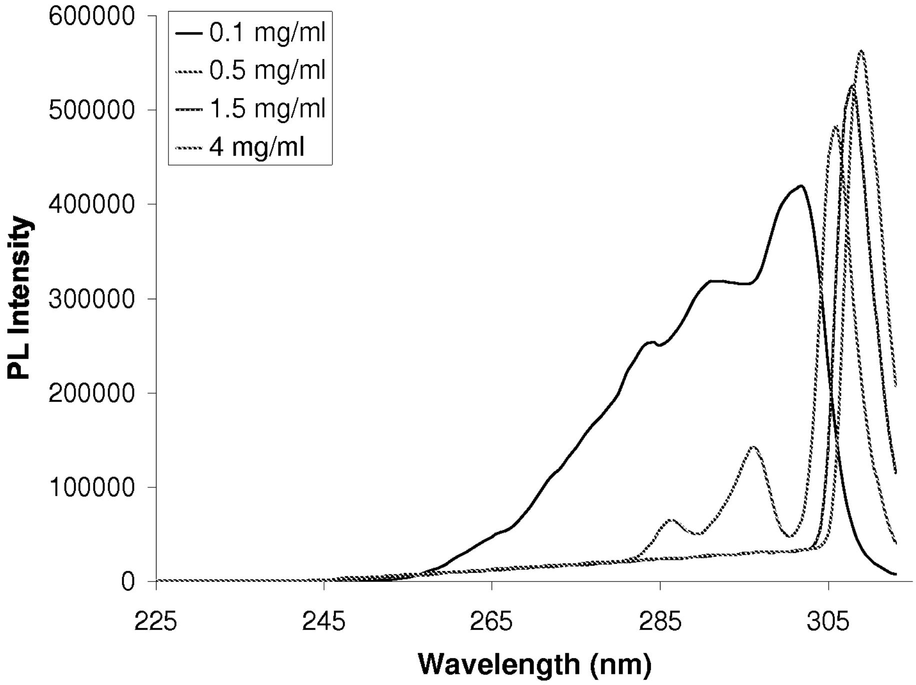
600000
500000
400000
300000
200000
100000
0
PL Intensity
0.1 mg/ml
0.5 mg/ml
1.5 mg/ml
4 mg/ml
225
245
265
285
305
Wavelength (nm)

FIG. 14

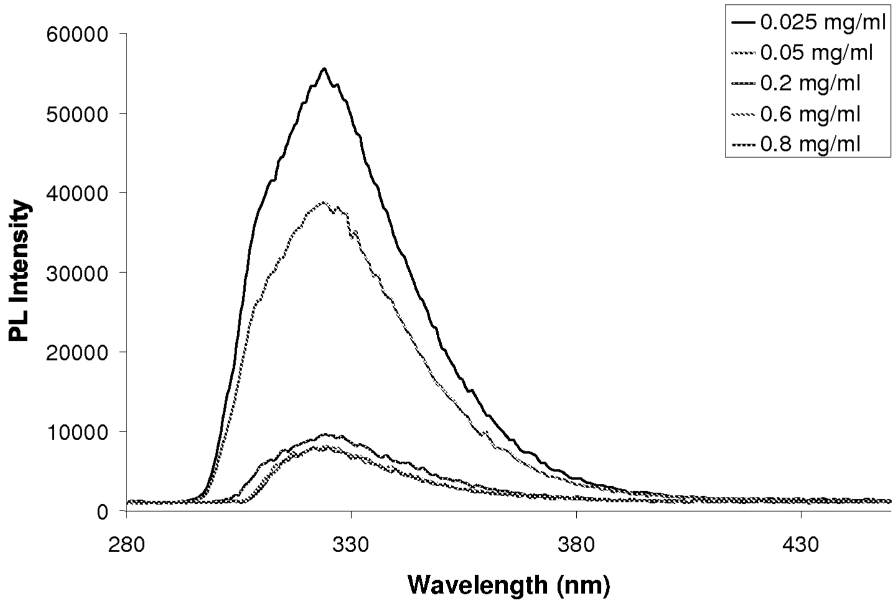


FIG. 15A

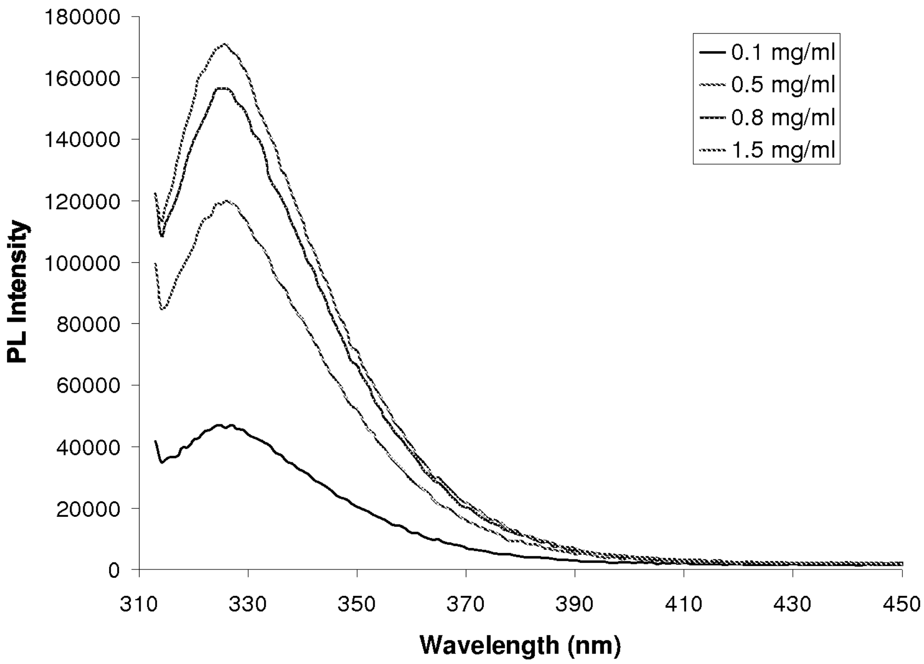


FIG. 15B

FIG. 16A

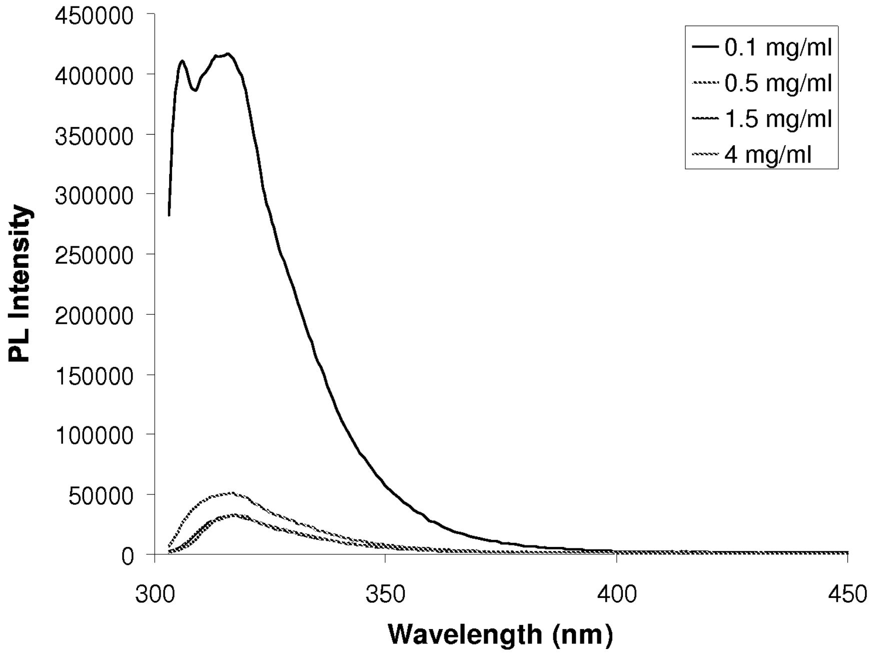


FIG. 16B

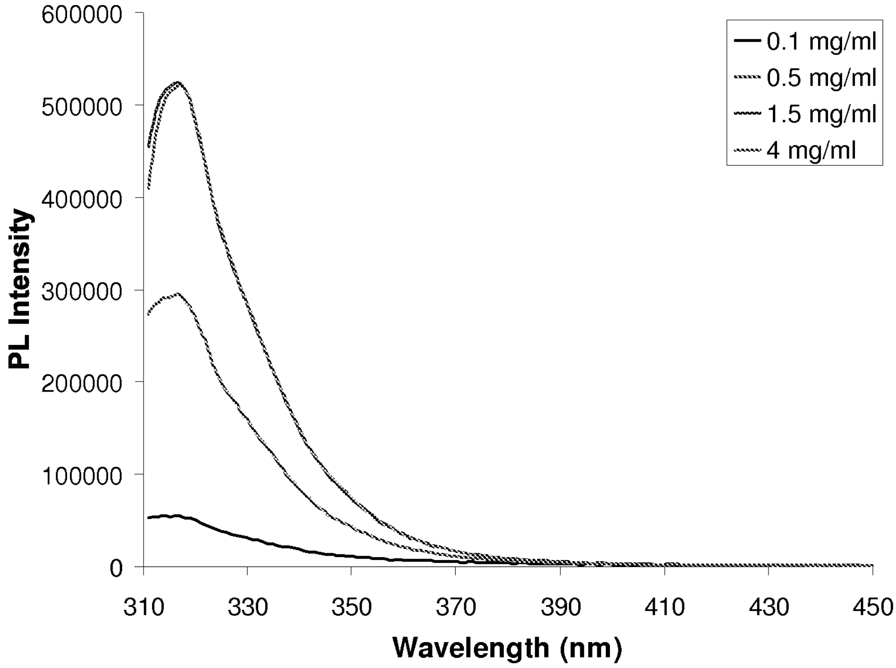

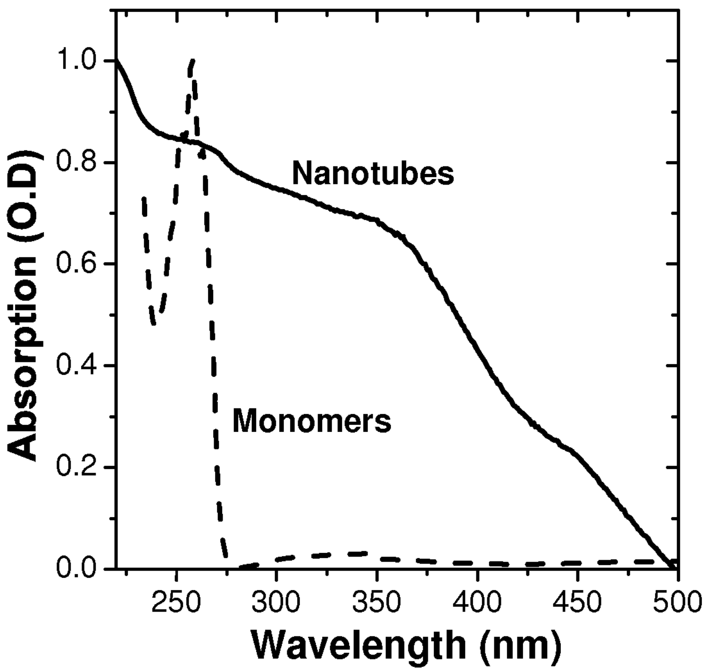
1.0
0.8
0.6
0.4
0.2
0.0
Absorption (O.D)
Nanotubes
Monomers
250
300
350
400
450
500
Wavelength (nm)

FIG. 17

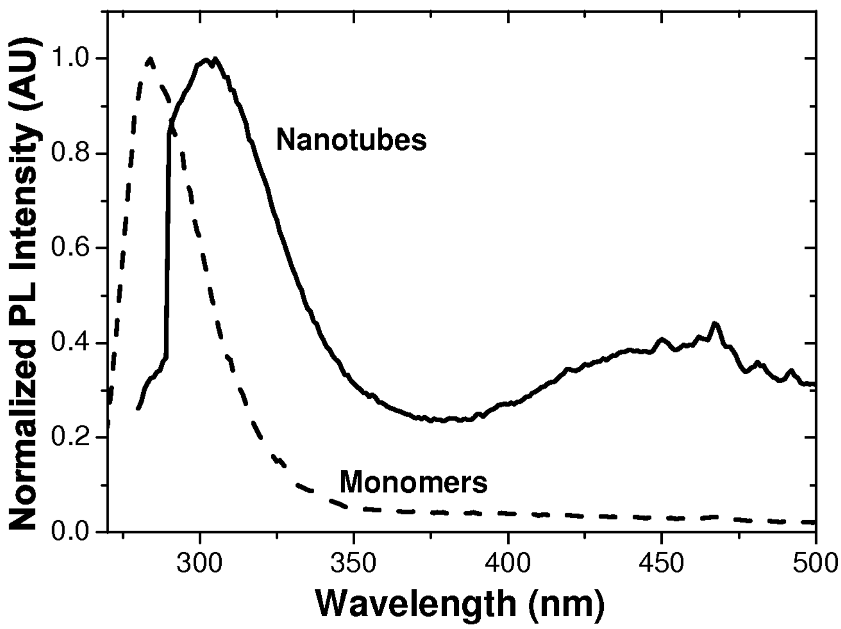
1.0
0.8
0.6
0.4
0.2
0.0
Normalized PL Intensity (AU)
Nanotubes
Monomers
300
350
400
450
500
Wavelength (nm)

FIG. 18

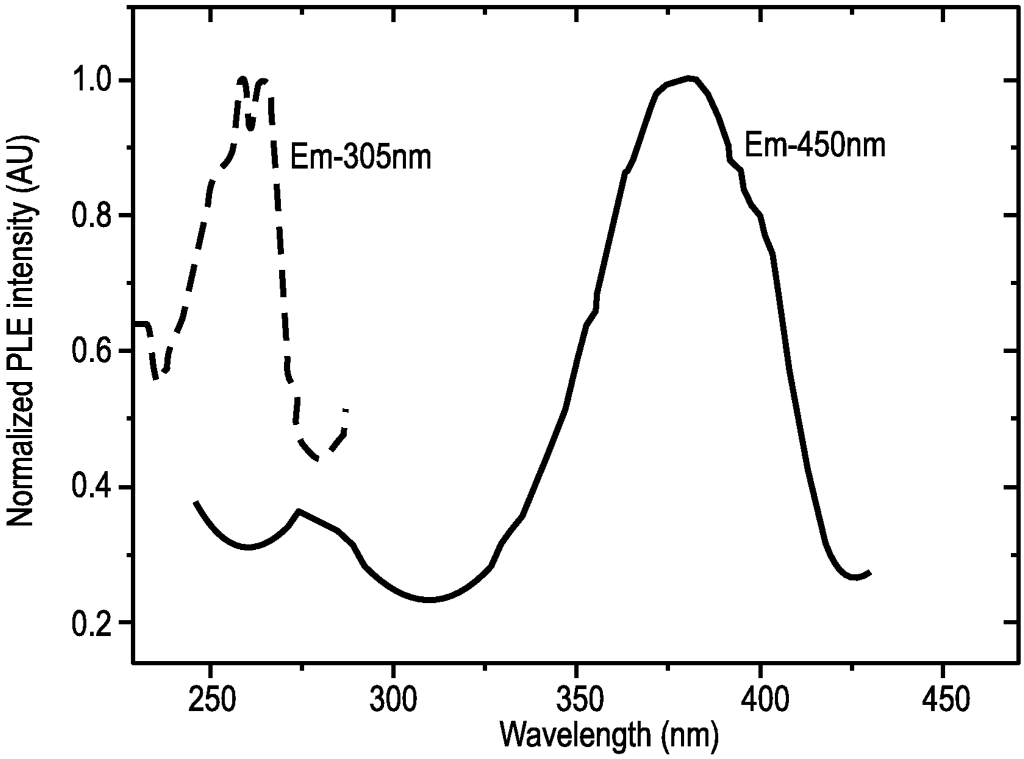


FIG. 19A

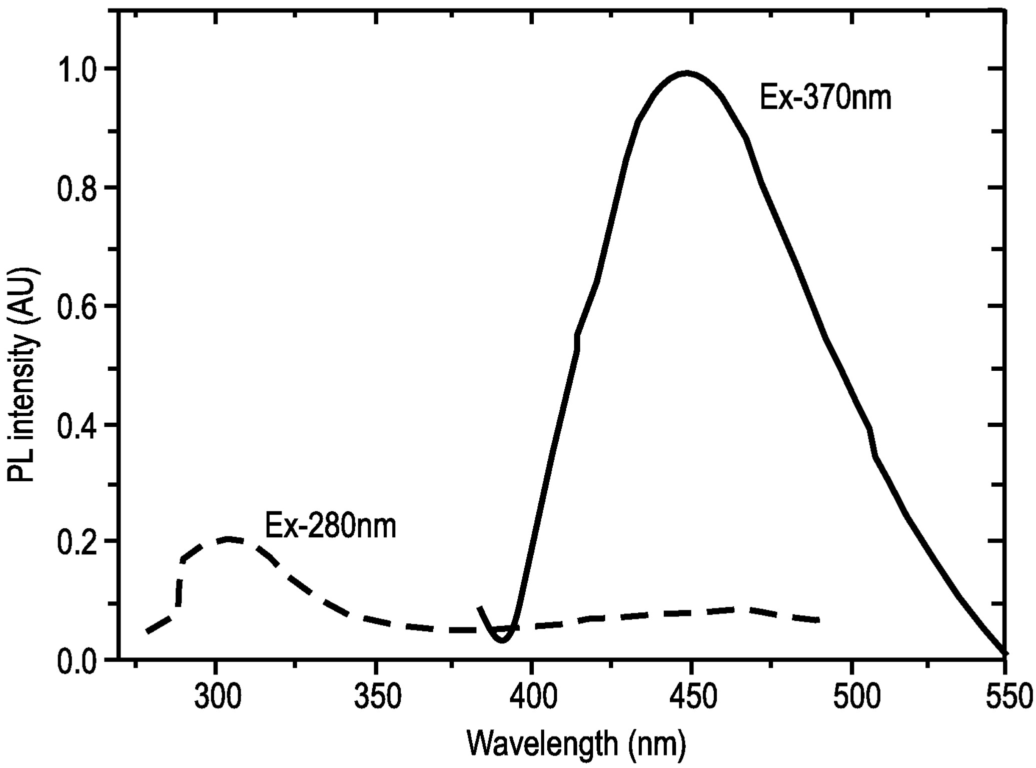


FIG. 19B

FIG. 20

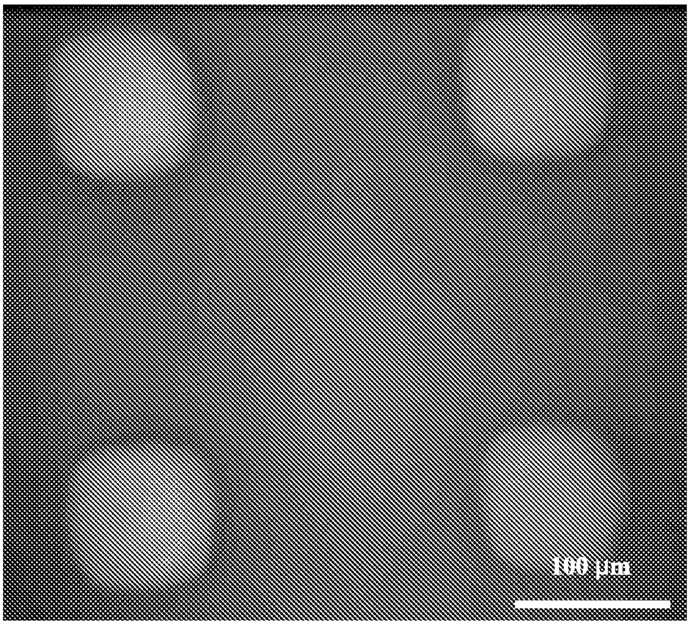


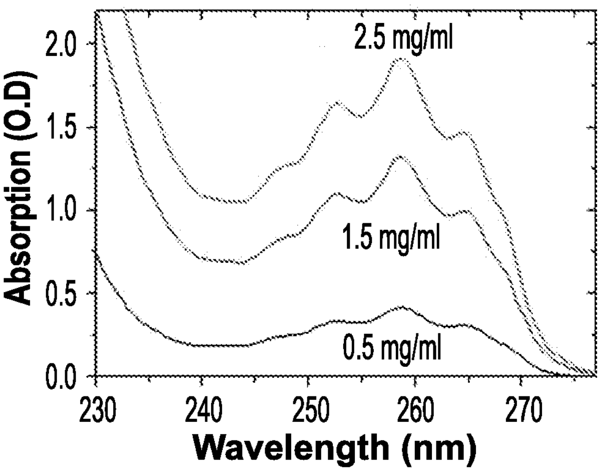


FIG. 21A

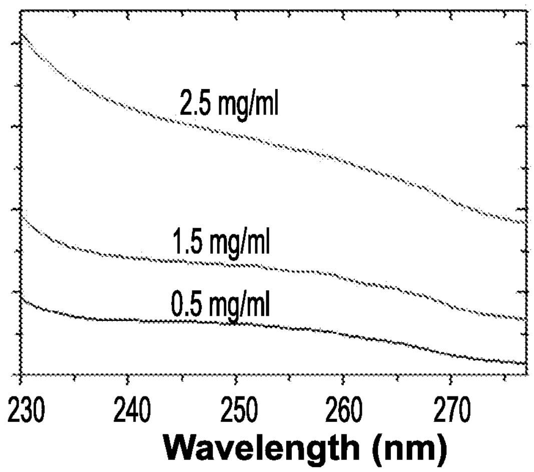


FIG. 21B

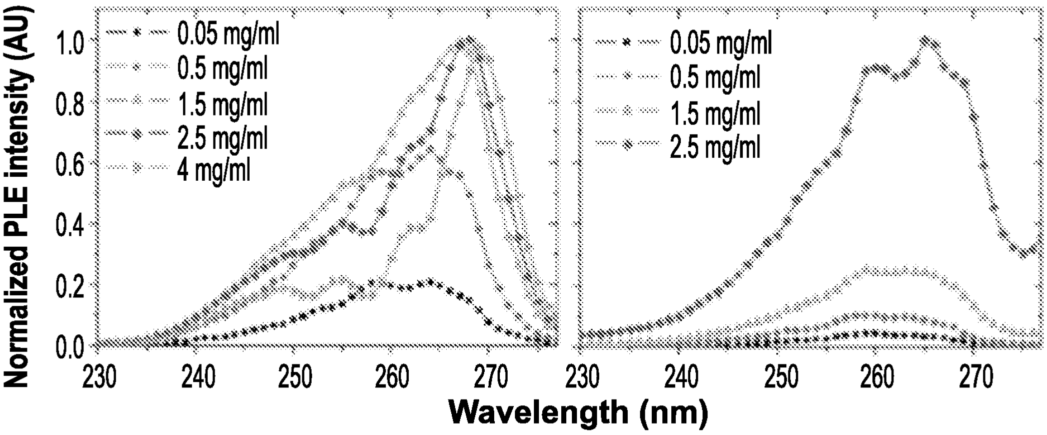


FIG. 22A FIG. 22B

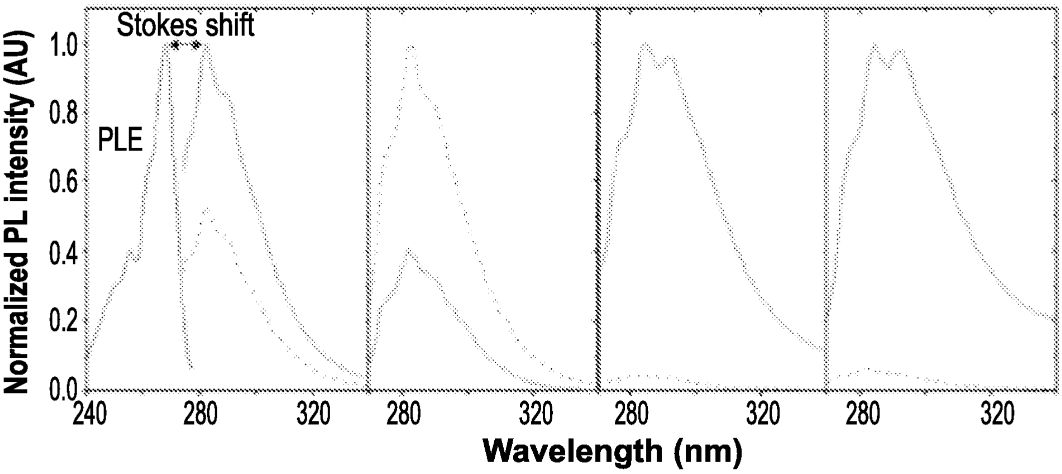


FIG. 23A FIG. 23B FIG. 23C FIG. 23D

FIG. 24A

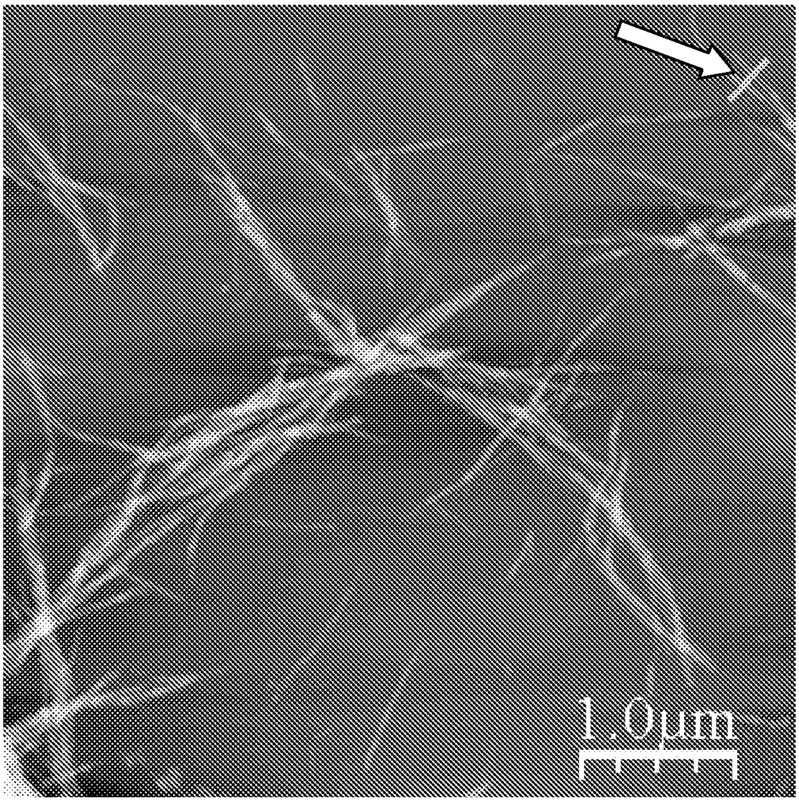


FIG. 24B

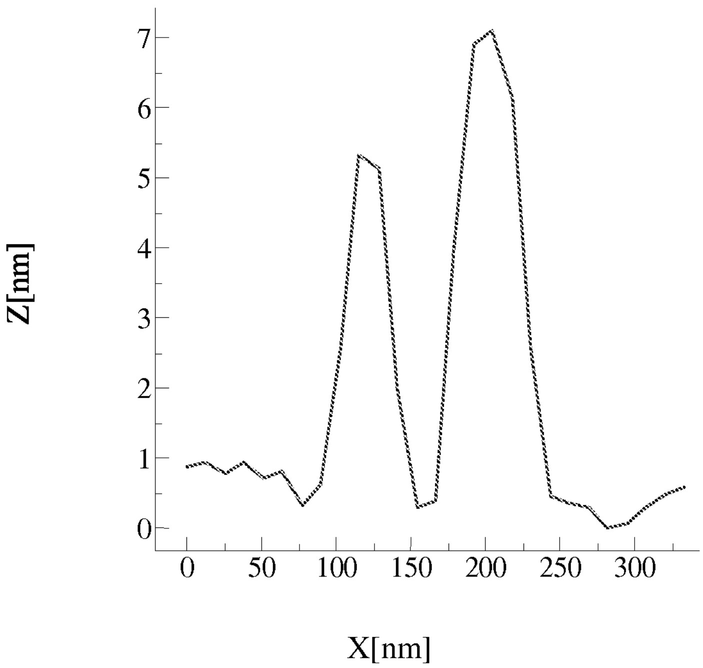

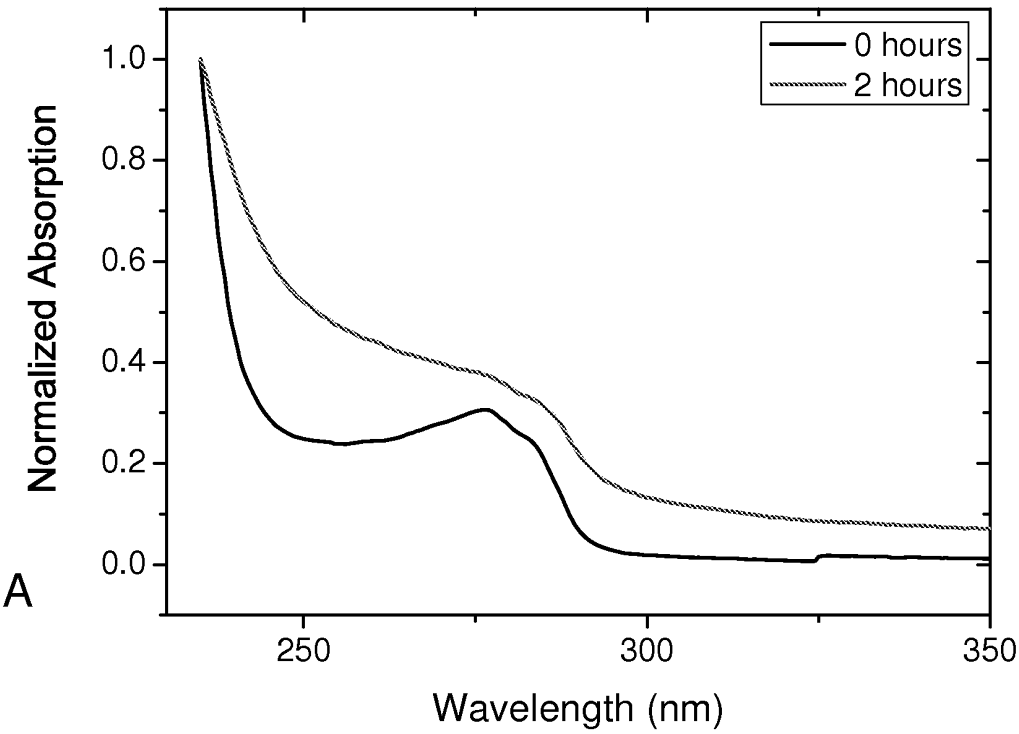


FIG. 25A

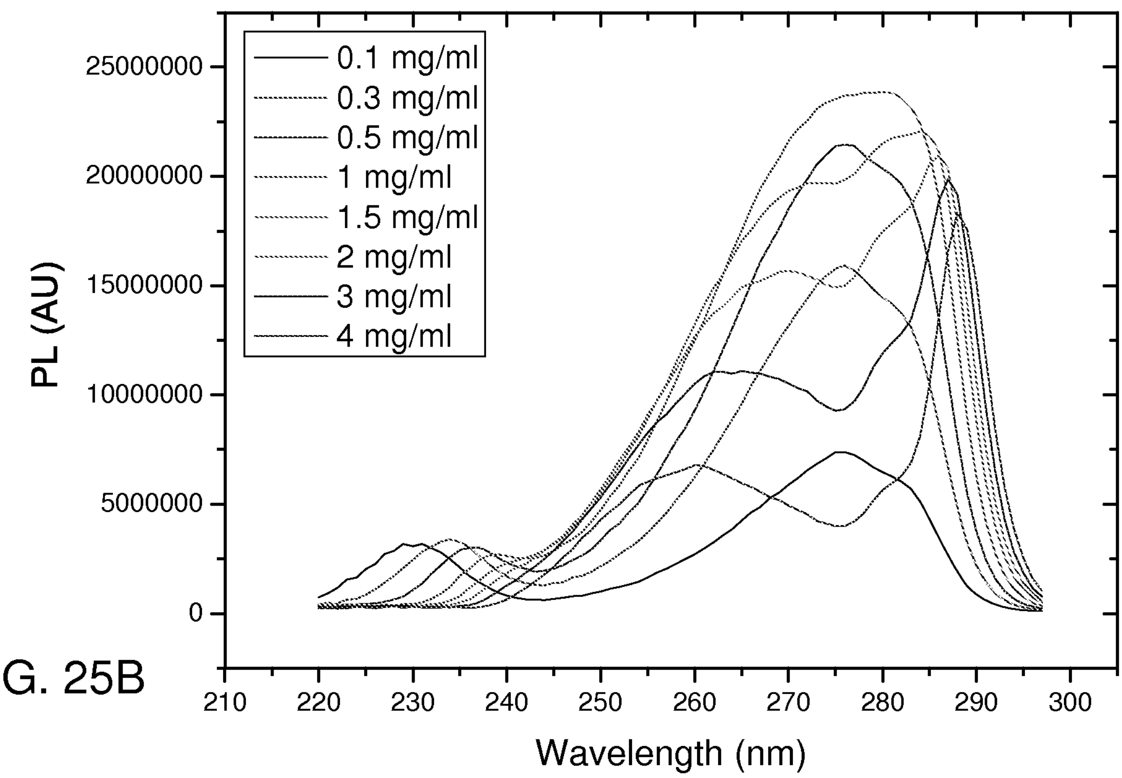


FIG. 25B

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 61136785 A
- US 61202758 A
- WO 2004052773 A
- WO 2004060791 A
- WO 2005000589 A
- WO 2006027780 A
- WO 2006013552 A
- WO 2008068752 A
- WO 2009034566 A
- WO 2007043048 A
- US 20020170590 A
- US 5912257 A
- US 5034613 A
- US 6020591 A
- US 595 A
- US 7960 A
- US 6267913 B
- US 5684621 A
- US 6605822 B
- US 5958883 A
- WO 2003063760 A
- WO 2005000193 A
- WO 2005027901 A
- WO 2006006172 A
- WO 2006018850 A


### Non-patent literature cited in the description


- **L. T. CANHAM.** Silicon quantum wire array fabrication by electrochemical and chemical dissolution of wafers. *AIP,* 1990, 1046-1048
- **PARK et al.** Quantum confinement observed in ZnO/ZnMgO nanorod heterostructures. *Adv. Mater.,* 2003, vol. 15, 526-529
- **TANAKA et al.** teach the use of self-assembled bionanostructures in polymer light-emitting diodes. *Nano Letters,* 2008, vol. 8 (9), 2S58-2861
- **DUKES et al.** teaches direct determination of reproducible distributions of peptide species by exploiting quantized photobleaching from individual dye-labeled Abeta peptides to characterize monomers and small oligomers tethered to the surface of functionalized coverslips. *Analytical Biochemistry,* 2008, vol. 382 (1), 29-34
- Quantitative Drug Design. Pergamon Press, 1992
- **GREEN et al.** Protective Groups in Organic Chemistry. Wiley, 1991
- **HARRISON et al.** Compendium of Synthetic Organic Methods. John Wiley and Sons, 1971, vol. 1-8
- **POPE, M. ; SWENBERG, C. E.** *Annu. Rev. Phys. Chem.,* 1984, vol. 35, 613-655
- **BASTARD et al.** *Phys. Rev. B,* 1982, vol. 26 (4), 1974-1979
- **POPE et al.** *Annu. Rev. Phys. Chem.,* 1984, vol. 35, 613-655
- **I. J. LALOV ; I. ZHELYAZKOV.** *Phys. Rev. B,* 2007, 75
- **C. D. ZANGMEISTER ; S. W. ROBEY ; R. D. VAN ZEE ; Y. YAO ; J. M. TOUR.** *J. Phys. Chem. B,* 2004, vol. 108, 16187